(19) Europäisches Patentamt
European Patent Office
Office européen des brevets

(11) EP 3 558 238 B1

# (12) EUROPEAN PATENT SPECIFICATION

(45) Date of publication and mention of the grant of the patent:
**23.08.2023 Bulletin 2023/34**

(21) Application number: **17811237.1**

(22) Date of filing: **24.11.2017**

(51) International Patent Classification (IPC):
***A61K 8/37*** (2006.01) ***A61K 8/40*** (2006.01)
***A61K 8/891*** (2006.01) ***A61K 8/29*** (2006.01)
***A61K 8/28*** (2006.01) ***A61K 8/19*** (2006.01)
***A61K 8/89*** (2006.01) ***A61Q 1/02*** (2006.01)
***A61Q 1/08*** (2006.01) ***A61Q 1/10*** (2006.01)

(52) Cooperative Patent Classification (CPC):
**A61K 8/37; A61K 8/19; A61K 8/28; A61K 8/29; A61K 8/40; A61K 8/89; A61K 8/891; A61Q 1/02; A61Q 1/08; A61Q 1/10**

(86) International application number:
**PCT/EP2017/080309**

(87) International publication number:
**WO 2018/114212 (28.06.2018 Gazette 2018/26)**

## (54) COMPOSITION CONTAINING AN OILY CONTINUOUS PHASE, A LIQUID DIPHENYLACRYLATE UV-SCREENING AGENT, A LIQUID SALICYLATE UV-SCREENING AGENT AND A NON-CYCLIC VOLATILE SILICONE OIL

ZUSAMMENSETZUNG MIT EINER KONTINUIERLICHEN ÖLPHASE, EINEM FLÜSSIGEN DIPHENYLACRYLAT-UV-SCHUTZMITTEL, EINEM FLÜSSIGEN SALICYLAT-UV-SCHUTZMITTEL UND EINEM NICHTZYKLISCHEN FLÜCHTIGEN SILIKONÖL

COMPOSITION CONTENANT UNE PHASE CONTINUE HUILEUSE, UN AGENT ANTI-UV LIQUIDE DIPHÉNYLACRYLATE, UN AGENT ANTI-UV LIQUIDE SALICYLATE ET UNE HUILE SILICONÉE VOLATILE NON CYCLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2016 FR 1662979**

(43) Date of publication of application:
**30.10.2019 Bulletin 2019/44**

(73) Proprietor: **L'OREAL**
**75008 Paris (FR)**

(72) Inventors:
- **VALVERDE, Elodie**
  **94152 Chevilly la Rue (FR)**
- **LI, Hong**
  **94152 Chevilly la Rue (FR)**

(74) Representative: **L'Oreal**
**Service D.I.P.I.**
**9, rue Pierre Dreyfus**
**92110 Clichy (FR)**

(56) References cited:

| | |
|---|---|
| **EP-A1- 2 100 584** | **WO-A1-03/013468** |
| **WO-A1-2015/082369** | **FR-A1- 2 861 593** |
| **FR-A1- 3 025 095** | **US-B1- 6 350 440** |

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 558 238 B1

## Description

The present invention is directed towards proposing for the field of caring for and/or making up keratin materials, especially the skin, a novel composition comprising an oily continuous phase and a mixture of particular liquid organic screening agents, which is most particularly advantageous with regard to its photoprotective performance and the sensations it affords the user during its application thereto and in particular to the skin.

Cosmetic makeup compositions, for example foundations, are commonly used to give keratin materials such as the skin an aesthetic colour, but also to enhance the beauty of irregular skin, by hiding marks and dyschromias, and to reduce the visibility of relief imperfections such as pores and wrinkles. Many formulations have been developed to date. A large amount of pigments is generally used in order to obtain a uniform and covering makeup result. The present invention is directed towards compositions with an oily continuous phase, especially in the form of a water-in-oil emulsion, which are particularly advantageous for their ease of formulation of pigments and their ease of application to keratin materials such as the skin.

Besides enhancing the complexion of the skin and correcting imperfections, it is also sought to protect keratin materials such as the skin on a daily basis against the effects induced by UV rays with a wavelength ranging from 280 to 400 nm. In particular, UV-B rays (wavelength of between 280 and 320 nm) may induce impairment of the biomechanical properties of the epidermis, which is reflected by the appearance of wrinkles, leading to premature ageing of the skin.

To remedy these effects induced by UVB rays, makeup compositions such as foundations, with an oily continuous phase, generally comprise at least lipophilic organic UV-screening agents which screen out at least the UVB radiation in amounts sufficient to be able to obtain good photoprotective efficacy which is reflected by a high sun protection factor SPF (for example SPF $\geq$ 30).

The sun protection factor (SPF) is expressed mathematically by the ratio of the UV radiation dose necessary to reach the erythema-forming threshold with the UV-screening agent to the UV radiation dose necessary to reach the erythema-forming threshold without UV-screening agent. This factor thus concerns the efficacy of the protection having a spectrum of biological action centred in the UVB range and consequently accounts for the protection with regard to this UVB radiation.

In these compositions, liquid organic UV-screening agents of the $\beta,\beta$-diphenylacrylate type such as the compound 2-ethylhexyl 2-cyano-3,3-diphenylacrylate or Octocrylene, sold especially under the trade name Uvinul N539 by BASF, are particularly advantageous for their miscibility in oils, their high efficacy with respect to UVB radiation and their good photostability. To obtain high protection factors, the use of these organic screening agents in large amounts in compositions with an oily continuous phase has a tendency to deteriorate their sensory qualities once applied to the skin, especially the non-tacky effect, the non-greasy effect, the soft feel, the sensation of lightness and the glidance.

Volatile silicone oils are commonly used in cosmetic compositions with an oily continuous phase for their good cosmetic properties, especially a pleasant feel on contact with the skin. These oils also evaporate rapidly after they have been applied to keratin materials. However, liquid organic UV-screening agents of the $\beta,\beta$-diphenylacrylate type are difficult to mix with volatile silicone oils and may lead to heterogeneous distribution of said screening agents on the skin and a loss of efficacy.

Patent applications WO 2010/045 163, US 2011/0 033 512 and WO 2007/148 293 disclose examples of a composition with an oily continuous phase comprising octocrylene, a UV-screening agent of the salicylate type, a combination of an emulsifying elastomeric silicone and a non-emulsifying elastomeric silicone, an emulsifier and a polar oil.

Patent application WO 2013/078 197 discloses examples of an antisun composition comprising a synergistic combination of five screening agents: avobenzone, homosalate, octylsalicylate, octocrylene and oxybenzone in specific weight ratios. These are silicone free alcoholic compositions or oil/water emulsions with silicone not specified.

Patent application US 2013/0 315 846 proposed a composition in the form of a very particular serum. Example 3 is a direct emulsion with a mixture of homosalate and octocrylene at 5% by weight in the presence of a mixture of volatile silicones DC 2-1184 at 1%.

Patent FR 2 861 593 also proposed examples of oil/water emulsion based on octocrylene, avobenzone and non-cyclic volatile silicone oils such as decamethyltetrasiloxane and dodecamethylpentasiloxane.

The need thus remains to find novel compositions for making up keratin materials, which have an oily continuous phase, which may contain at least one organic UV-screening agent of the $\beta,\beta$-diphenylacrylate type in large amounts in the presence of at least one volatile silicone oil and pigments, which gives good photoprotective efficacy, good makeup qualities such as coverage and good homogeneity of the complexion and also good sensory qualities such as softness, a non-tacky effect, a non-greasy effect, the absence of an unpleasant film sensation after application, a sensation of lightness after application and good glidance, without the technical drawbacks listed previously.

The Applicant has discovered, surprisingly, that this objective can be achieved with a composition, especially comprising a physiologically acceptable medium, especially for coating keratin materials, more particularly for making up and/or caring for keratin materials such as the skin, containing at least:

a) an oily continuous phase, and
b) at least one mixture of liquid organic UV-screening agents comprising:

(i) at least one UV-screening agent of the β,β-diphenylacrylate type, and
(ii) at least one UV-screening agent of the salicylate type;

c) at least one non-cyclic volatile silicone oil; and
d) at least one pigment; **the said composition being**

**i) an anhydrous composition comprising a single oily phase such as an oil, an oily gel or an anhydrous cream, or**
**ii) a water-in-oil emulsion**

This discovery forms the basis of the invention.

According to one of its aspects, the present invention relates to a composition, especially comprising a physiologically acceptable medium, especially for coating keratin materials, more particularly for making up and/or caring for keratin materials such as the skin,

a) an oily continuous phase, and
b) at least one mixture of liquid organic UV-screening agents comprising:

(i) at least one UV-screening agent of the β,β-diphenylacrylate type, and
(ii) at least one UV-screening agent of the salicylate type; and

c) at least one non-cyclic volatile silicone oil; and
d) at least one pigment; **the said composition being**

**i) an anhydrous composition comprising a single oily phase such as an oil, an oily gel or an anhydrous cream, or**
**ii) a water-in-oil emulsion**

The invention also relates to a process for coating keratin materials, more particularly for making up and/or caring for keratin materials, such as the skin, characterized in that it comprises the application to the keratin materials of a composition as defined previously.

## DEFINITIONS

In the context of the present invention, the term "keratin material" especially means the skin (of the body, face, around the eyes, or the eyelids).

The term "physiologically acceptable" means compatible with the skin and/or its integuments, which has a pleasant colour, odour and feel, and which does not cause any unacceptable discomfort (stinging or tautness) liable to discourage the consumer from using this composition.

The term "composition containing an oily continuous phase" means

i) an anhydrous composition comprising a single oily phase such as an oil, an oily gel or an anhydrous cream,
ii) or a water-in-oil emulsion, also known as an inverse emulsion.

For the purposes of the present invention, the term "anhydrous" refers to a composition comprising a content of less than or equal to 2% by weight, preferably less than or equal to 1% and more preferentially less than 0.5% by weight of water relative to the total weight of said composition, or is even free of water. Where appropriate, such small amounts of water may especially be introduced by ingredients of the composition that may contain residual amounts thereof.

For the purposes of the present invention, the term "water-in-oil emulsion", also referred to as inverse emulsion, is intended to denote any composition constituted of an oily continuous phase in which the aqueous phase is dispersed in the form of droplets so as to observe a macroscopically homogeneous mixture with the naked eye.

The term "liquid organic UV-screening agent" means any organic compound which is capable of filtering out UV radiation ranging from 280 to 400 nm and which is liquid at room temperature (20-25°C) and atmospheric pressure.

## MIXTURE OF 6.6-DIPHENYLACRYLATE/SALICYLATE LIQUID ORGANIC UV-SCREENING AGENTS

The composition according to the invention comprises a mixture of liquid organic UV-screening agents comprising:

(i) at least one UV-screening agent of the β,β-diphenylacrylate type, and
(ii) at least one UV-screening agent of the salicylate type.

According to a preferential form of the invention, said mixture of screening agents will be present in the composition of the invention in a concentration of at least 15% by weight, and more preferentially in a concentration of at least 20% by weight, relative to the total weight of the composition.

Preferably, said mixture of β,β-diphenylacrylate/salicylate screening agents is used in concentrations ranging from 15% to 40% by weight and more preferentially ranging from 20% to 30% by weight relative to the total weight of the composition.

### a) β,β-Diphenylacrylate compounds

Among the UV-screening agents of the β,β-diphenylacrylate type, mention may be made of those of formula (I) below:

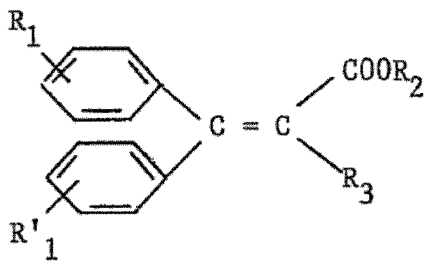

(I)

in which $R_1$ to $R_3$ can have the following meanings:

- $R_1$ and $R'_1$, which may be identical or different, represent a hydrogen atom, a straight-chain or branched-chain $C_1$-$C_8$ alkoxy radical or a straight-chain or branched-chain $C_1$-$C_4$ alkyl radical;
- $R_1$ and $R'_1$ being in the para or meta position;
- $R_2$ represents a straight-chain or branched-chain $C_1$-$C_{12}$ alkyl radical;
- $R_3$ represents a hydrogen atom or the CN radical.

Among the straight-chain or branched-chain $C_1$-$C_8$ alkoxy radicals, mention may be made, for example, of methoxy, ethoxy, n-propoxy, isopropoxy, n-butoxy, isobutoxy, tert-butoxy, n-amyloxy, isoamyloxy, neopentyloxy, n-hexyloxy, n-heptyloxy, n-octyloxy and 2-ethylhexyloxy radicals.

Among the straight-chain or branched-chain $C_1$-$C_4$ alkyl radicals, mention may more particularly be made of methyl, ethyl, n-propyl, isopropyl, n-butyl, isobutyl and tert-butyl radicals. For the $C_1$-$C_{12}$ alkyl radicals, mention may be made, by way of example, in addition to those mentioned above, of n-amyl, isoamyl, neopentyl, n-hexyl, n-heptyl, n-octyl, 2-ethylhexyl, decyl and lauryl radicals.

Among the compounds of general formula (I), mention may be made of the following compounds:

2-ethylhexyl α-cyano-β,β-diphenylacrylate, or Octocrylene, sold especially under the trade name Uvinul N539 by BASF,
ethyl α-cyano-β,β-diphenylacrylate, such as Etocrylene, sold especially under the trade name Uvinul N35@ by BASF,
2-ethylhexyl β,β-diphenylacrylate,
ethyl β,β-bis(4'-methoxyphenyl)acrylate.

Among the UV-screening agents of the β,β-diphenylacrylate type, use will be made more particularly of the compound 2-ethylhexyl 2-cyano-3,3-diphenylacrylate, or Octocrylene, sold especially under the trade name Uvinul N539 by BASF.

The β,β-diphenylacrylate compound(s) are preferably present in the composition in concentrations ranging from 5% to 10% and more preferentially from 6% to 8% by weight relative to the total weight of the composition.

**b) Salicylate compounds**

Among the salicylate compounds that may be used according to the invention, use will be made of:

- Homosalate sold under the name Eusolex HMS by Rona/EM Industries,
- Ethylhexyl salicylate sold under the name Neo Heliopan OS by Symrise,
- and mixtures thereof.

The salicylate compound(s) are preferably present in the composition in concentrations ranging from 5% to 20% and more preferentially from 10% to 15% by weight relative to the total weight of the composition.

**OILY CONTINUOUS PHASE**

The composition of the invention comprises an oily continuous phase. Said phase is liquid (in the absence of structuring agent) at room temperature (20-25°C). It is organic and water-immiscible.

It comprises at least one non-cyclic volatile silicone oil and the mixture of β,β-diphenylacrylate/salicylate liquid organic UV-screening agents.

It may also contain one or more additional oils that may be chosen from

- cyclic volatile silicone oils;
- non-volatile oils chosen from hydrocarbon-based oils, silicone oils and fluoro oils, and mixtures thereof,
- volatile hydrocarbon-based oils;
- ingredients that are soluble or miscible in the oily phase;
- mixtures thereof.

The term "oil" means a fatty substance that is liquid at room temperature (25°C) and atmospheric pressure (760 mmHg, i.e. $10^5$ Pa). The oil may be volatile or non-volatile.

For the purposes of the present invention, the term "silicone oil" means an oil comprising at least one silicon atom, and especially at least one Si-O group, and more particularly an organopolysiloxane.

The term "fluoro oil" means an oil comprising at least one fluorine atom.

The term "hydrocarbon-based oil" means an oil mainly containing carbon and hydrogen atoms and possibly one or more functions chosen from hydroxyl, ester, ether and carboxylic functions.

The term *"non-cyclic* oil" denotes a linear or branched oil, not comprising any rings. In other words, the oil in accordance with the invention is free of (hetero)aryl radicals and/or of (hetero)cycloalkyl radicals.

For the purposes of the invention, the term "volatile oil" means any oil that is capable of evaporating on contact with the skin in less than one hour, at room temperature and atmospheric pressure. The volatile oil is a volatile cosmetic compound, which is liquid at room temperature, especially having a non-zero vapour pressure, at room temperature and atmospheric pressure, especially having a vapour pressure ranging from 0.13 Pa to 40 000 Pa ($10^{-3}$ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" means an oil that remains on the skin or the keratin fibre at room temperature and atmospheric pressure for at least several hours, and that especially has a vapour pressure of less than $10^{-3}$ mmHg (0.13 Pa).

The total concentration of oily phase in the composition of the invention preferably ranges from 30% to 95% by weight and more particularly ranging from 40% to 70% by weight relative to the total weight of the composition.

**A) Non-cyclic volatile silicone oils**

According to the invention, the non-cyclic volatile silicone oils according to the invention are preferably chosen from:

- the non-cyclic linear silicones of formula (II):

$$R_3SiO-(R_2SiO)_n-SiR_3 \qquad (II)$$

in which R, which may be identical or different, denotes:

- a saturated or unsaturated hydrocarbon-based radical, containing from 1 to 10 carbon atoms and preferably from 1 to 6 carbon atoms, optionally substituted with one or more fluorine atoms or with one or more hydroxyl groups, or
- a hydroxyl group,

one of the radicals R possibly being a phenyl group,
n is an integer ranging from 0 to 8, preferably ranging from 2 to 6 and better still ranging from 3 to 5, the silicone compound of formula (II) containing not more than 15 carbon atoms,

- the branched silicones of formula (III) or (IV) below:

$$R_3SiO\text{-}[(R_3SiO)RSiO]\text{-}(R_2SiO)x\text{-}SiR_3 \qquad (III)$$

$$[R_3SiO]_4Si \qquad (IV)$$

in which R, which may be identical or different, denotes:

- a saturated or unsaturated hydrocarbon-based radical, containing from 1 to 10 carbon atoms, optionally substituted with one or more fluorine atoms or with one or more hydroxyl groups, or
- a hydroxyl group,

one of the radicals R possibly being a phenyl group,
x is an integer ranging from 0 to 8,
the silicone compound of formula (III) or (IV) containing not more than 15 carbon atoms.

Preferably, for the compounds of formulae (II), (III) and (IV), the ratio between the number of carbon atoms and the number of silicon atoms is between 2.25 and 4.33.
The silicones of formulae (II) to (IV) may be prepared according to the known processes for synthesizing silicone compounds.
Examples of non-cyclic volatile silicones that may be used according to the invention are indicated below; these silicones may be used alone or as a mixture.
Among the silicones of formula (II) that may be mentioned are:

a) The disiloxanes (L2) below:

hexamethyldisiloxane, sold especially under the name DC 200 Fluid 0.65 cst by the company Dow Corning
1 ,3-di-tert-butyl-1, 1 ,3,3-tetramethyldisiloxane;
1,3-dipropyl-1, 1,3,3-tetramethyldisiloxane-, heptylpentamethyldisiloxane;
1,1,1-triethyl-3,3,3-trimethyldisiloxane;
hexaethyldisiloxane;
1,1,3,3-tetramethyl-1,3-bis(2-methylpropyl)disiloxane;
pentamethyloctyldisiloxane;
1,1,1-trimethyl-3,3,3-tris(1-methylethyl)disiloxane;
1-butyl-3-ethyl-1,1,3-trimethyl-3-propyldisiloxane;
pentamethylpentyldisiloxane;
1-butyl-1,1,3,3-tetramethyl-3-(1-methylethyl)disiloxane;
1,1,3,3-tetramethyl-1,3-bis(1-methylpropyl)disiloxane;
1,1,3-triethyl-1,3,3-tripropyldisiloxane;
(3,3-dimethylbutyl)pentamethyldisiloxane;
(3-methylbutyl)pentamethyldisiloxane;
(3-methylpentyl)pentamethyldisiloxane;
1,1,1-triethyl-3,3-dimethyl-3-propyldisiloxane;
1-(1,1-dimethylethyl)-1,1,3,3,3-pentamethyldisiloxane;
1,1,1-trimethyl-3,3,3-tripropyldisiloxane;
1,3-dimethyl-1,1,3,3-tetrakis(1-methylethyl)disiloxane;
1,1-dibutyl-1,3,3,3-tetramethyldisiloxane;
1,1,3,3-tetramethyl-1,3-bis(1-methylethyl)disiloxane;
1,1,1,3-tetramethyl-3,3-bis(1-methylethyl)disiloxane;

1,1,1,3-tetramethyl-3,3-dipropyldisiloxane;
1,1,3,3-tetramethyl-1,3-bis(3-methylbutyl)disiloxane;
butylpentamethyldisiloxane;
pentaethylmethyldisiloxane;
1,1,3,3-tetramethyl-1,3-dipentyldisiloxane;
1 ,3-dimethyl-1, 1 ,3,3-tetrapropyldisiloxane;
1,1,1,3-tetraethyl-3,3-dimethyldisiloxane;
1,1,1-triethyl-3,3,3-tripropyldisiloxane;
1,3-dibutyl-1, 1,3,3-tetramethyldisiloxane-, hexylpentamethyldisiloxane;

b) the trisiloxanes (L3) below:

octamethyltrisiloxane, sold especially under the name Xiameter PMX-200 Silicone Fluid 1CS by the company Dow Corning;
3-pentyl-1,1,1,3,5,5,5-heptamethyltrisiloxane;
1-hexyl-1,1,3,3,5,5,5-heptamethyltrisiloxane;
1,1,1,3,3,5,5-heptamethyl-5-octyltrisiloxane;
1,1,1,3,5,5,5-heptamethyl-3-octyltrisiloxane, sold especially under the name Silsoft 034 by the company OSI;
1,1,1,3,5,5,5-heptamethyl-3-hexyltrisiloxane, sold especially under the name DC 2-1731 by the company Dow Corning;
1,1,3,3,5,5-hexamethyl-1,5-dipropyltrisiloxane;
3-(1-ethylbutyl)-1,1,1, 3, 5, 5, 5-heptamethyltrisiloxane;
1,1,1,3,5,5,5-heptamethyl-3-(1-methylpentyl)trisiloxane;
1,5-diethyl-1,1,3,3,5,5-hexamethyltrisiloxane;
1,1,1,3,5,5,5-heptamethyl-3-(1-methylpropyl)trisiloxane;
3-(1,1-dimethylethyl)-1,1,1,3,5,5,5-heptamethyltrisiloxane;
1,1,1,5,5,5-hexamethyl-3,3-bis(1-methylethyl)trisiloxane;
1,1,1,3,3,5,5-heptamethyl-1,5-bis(1-methylpropyl)trisiloxane;
1,5-bis(1,1-dimethylethyl)-1,1,3,3,5,5-hexamethyltrisiloxane;
3-(3,3-dimethylbutyl)-1,1,1,3,5,5,5-heptamethyltrisiloxane;
1,1,1,3,5,5,5-heptamethyl-3-(3-methylbutyl)trisiloxane;
1,1,1,3,5,5,5-heptamethyl-3-(3-methylpentyl)trisiloxane;
1,1,1,3,5,5,5-heptamethyl-3-(2-methylpropyl)trisiloxane;
1-butyl-1,1,3,3,5,5,5-heptamethyltrisiloxane;
1,1,1,3,5,5,5-heptamethyl-3-propyltrisiloxane;
3-isohexyl-1,1,1,3,5,5,5-heptamethyltrisiloxane;
1,3,5-triethyl-1,1,3,5,5-pentamethyltrisiloxane;
3-butyl-1,1,1,3,5,5,5-heptamethyltrisiloxane;
3-tert-pentyl-1,1,1,3,5,5,5-heptamethyltrisiloxane;
1,1,1,5,5,5-hexamethyl-3,3-dipropyltrisiloxane;
3,3-diethyl-1,1,1,5,5,5-hexamethyltrisiloxane;
1,5-dibutyl-1,1,3,3,5,5-hexamethyltrisiloxane;
1,1,1,5,5,5-hexaethyl-3,3-dimethyltrisiloxane;
3,3-dibutyl-1,1,1,5,5,5-hexamethyltrisiloxane;
3-ethyl-1,1,1,3,5,5,5-heptamethyltrisiloxane;
3-heptyl-1,1,1,3,5,5,5-heptamethyltrisiloxane;
1-ethyl-1,1,3,3,5,5,5-heptamethyltrisiloxane;

c) the tetrasiloxanes (L4) below:

decamethyltetrasiloxane;
1,1,3,3,5,5,7,7-octamethyl-1,7-dipropyltetrasiloxane;
1,1,1,3,3,5,7,7,7-nonamethyl-5-(1-methylethyl)tetrasiloxane;
1-butyl-1,1, 3, 3, 5, 5, 7, 7, 7-nonamethyltetrasiloxane;
3,5-diethyl-1,1,1,3,5,7,7,7-octamethyltetrasiloxane;
1,3,5,7-tetraethyl-1,1,3,5,7,7-hexamethyltetrasiloxane;
3,3,5,5-tetraethyl-1,1,1,7,7,7-hexamethyltetrasiloxane;
1,1,1,3,3,5,5,7,7-nonamethyl-7-phenyltetrasiloxane;

3,3-diethyl-1,1,1,5,5,7,7,7-octamethyltetrasiloxane;
1,1,1,3,3,5,7,7,7-nonamethyl-5-phenyltetrasiloxane;

d) the pentasiloxanes (L5) below:

dodecamethylpentasiloxane;
1,1,3,3,5,5,7,7,9,9-decamethyl-1,9-dipropylpentasiloxane;
3,3,5,5,7,7-hexaethyl-1,1,1,9,9,9-hexamethylpentasiloxane;
1,1,1,3,3,5,7,7,9,9,9-undecamethyl-5-phenylpentasiloxane;
1-butyl-1,1,3,3,5,5,7,7,9,9,9-undecamethylpentasiloxane;
3,3-diethyl-1,1,1,5,5,7,7,9,9,9-decamethylpentasiloxane;
1,3,5,7,9-pentaethyl-1,1,3,5,7,9,9-heptamethylpentasiloxane;
3,5,7-triethyl-1,1,1,3,5,7,9,9,9-nonamethylpentasiloxane;
1,1,1-triethyl-3,3,5,5,7,7,9,9,9-nonamethylpentasiloxane

e) the hexasiloxanes (L6) below:
1-butyl-1,1,3,3,5,5,7,7,9,9,11,11,11-tridecamethylhexasiloxane; 3,5,7,9-tetraethyl-1,1,1,3,5,7,9,11,11,11-decamethylhexasiloxane; tetradecamethylhexasiloxane;

f) hexadecamethylheptasiloxane (L7);

g) octadecamethyloctasiloxane (L8).

Among the silicones of formula (III), mention may be made of:

a) the tetrasiloxanes (L4) below:

2-[3,3,3-trimethyl-1,1-bis[(trimethylsilyl)oxy]disiloxanyl]ethyl;
1,1,1,5,5,5-hexamethyl-3-(2-methylpropyl)-3-[(trimethylsilyl)oxy]trisiloxane;
3-(1,1-dimethylethyl)-1,1,1,5,5,5-hexamethyl-3-[(trimethylsilyl)oxy]trisiloxane;
3-butyl-1,1,1,5,5,5-hexamethyl-3-[(trimethylsilyl)oxy]trisiloxane;
1,1,1,5,5,5-hexamethyl-3-propyl-3-[(trimethylsilyl)oxy]trisiloxane;
3-ethyl-1,1,1,5,5,5-hexamethyl-3-[(trimethylsilyl)oxy]trisiloxane;
1,1,1-triethyl-3,5,5,5-tetramethyl-3-(trimethylsiloxy)trisiloxane;
3-methyl-1,1,1,5,5,5-hexamethyl-3-[trimethylsilyl)oxy]trisiloxane;
3-[(dimethylphenylsilyl)oxy]-1,1,1,3,5,5,5-heptamethyltrisiloxane;
1,1,1,5,5,5-hexamethyl-3-(2-methylpentyl)-3-[(trimethylsilyl)oxy]trisiloxane;
1,1,1,5,5,5-hexamethyl-3-(4-methylpentyl)-3-[(trimethylsilyl)oxy]trisiloxane;
3-hexyl-1,1,1,5,5,5-hexamethyl-3-[(trimethylsilyl)oxy]trisiloxane;
1,1,1,3,5,5,5-heptamethyl-3-[(trimethylsilyl)oxy]trisiloxane;

b) the pentasiloxanes (L5) below:

1,1,1, 3, 5, 5, 7, 7, 7-nonamethyl-3-(trim ethylsi loxy)tetrasi loxane;
1,1,1, 3, 3, 7, 7, 7-octam ethyl-5-phenyl-5-[(trim ethylsi lyl)oxy]tetrasi loxane;

c) the heptasiloxanes (L7) below: 1,1,1,3,5,5,7,7,9,9,11,11,11-tridecamethyl-3-[(trimethylsilyl)oxy]hexasiloxane.

Among the silicones of formula (IV), mention may be made of: 1,1,1,5,5,5-hexamethyl-3,3-bis(trimethylsiloxy)trisiloxane.

Use may also be made of other volatile silicone oils chosen from:

a) the tetrasiloxanes (L4) below:

2,2,8,8-tetramethyl-5-[(pentamethyldisiloxanyl)methyl]-3,7-dioxa-2,8-disilanonane;
2,2,5,8,8-pentamethyl-5-[(trimethylsilyl)methoxy]-4,6-dioxa-2,5,8-trisilanonane;
1,3-dimethyl-1,3-bis[(trimethylsilyl)methyl]-1,3-disiloxanediol;
3-ethyl-1,1,1,5,5,5-hexamethyl-3-[3-(trimethylsiloxy)propyl]trisiloxane;

1,1,1,5,5,5-hexamethyl-3-phenyl-3-[(trimethylsilyl)oxy]trisiloxane (Dow 556 Fluid);

b) the pentasiloxanes (L5) below:

2,2,7,7,9,9,11,11,16,16-decamethyl-3,8,10,15-tetraoxa-2,7,9,11,16-pentasilaheptadecane;
silicic acid tetrakis[(trimethylsilyl)methyl] ester;

c) the hexasiloxanes (L6) below:
3,5-diethyl-1,1,1,7,7,7-hexamethyl-3,5-bis[(trimethylsilyl)oxy]tetrasiloxane, 1,1,1,3,5,7,7,7-octamethyl-3,5-bis[(trimethylsilyl)oxy]tetrasiloxane;

d) the heptasiloxane (L7):
1,1,1, 3, 7, 7, 7-heptam ethyl-3, 5, 5-tris[(trimethylsi lyl)oxy]tetrasi loxane;

e) the octasiloxanes (L8) below:

1,1,1,3,5,5,9,9,9-nonamethyl-3,7,7-tris[(trimethylsilyl)oxy]pentasiloxane-,
1,1,1, 3, 5, 7, 9, 9, 9-nonamethyl-3, 5, 7-tris[(trimethylsi lyl)oxy]pentasi loxane;
1,1,1,7,7,7-hexamethyl-3,3,5,5-tetrakis[(trimethylsilyl)oxy]tetrasiloxane.

The non-cyclic volatile silicone oils according to the invention preferably have a viscosity at 25°C ranging from 0.5 to 8 centistokes (from 0.5 to 8 $mm^2/s$). The viscosity measurement method used in the invention to characterize the silicone oils according to the invention may be the "kinematic viscosity at 25°C raw product CID-012-01" or the "Ubbelohde viscosity at 25°C DIN 51562-1 PV04001".

Among the non-cyclic volatile silicone oils according to the invention, linear non-cyclic volatile silicone oils will more particularly be chosen, and more particularly:

- octamethyltrisiloxane, sold especially under the name Xiameter PMX-200 Silicone Fluid 1CS by the company Dow Corning;
- decamethyltetrasiloxane, sold especially under the name Xiameter PMX-200 Silicone Fluid 1.5CS® by the company Dow Corning;
- dodecamethylpentasiloxane, such as the commercial products sold under the names KF-96L-2CS®, DM-Fluid-2CS® by Shin Etsu; Berb- DM2® by BRB International or Silicone Fluid 2CS® by Dow Corning;
- mixtures thereof, and more particularly dodecamethylpentasiloxane.

According to a preferential form of the invention, the non-cyclic volatile silicone oil/mixture of β,β-diphenylacrylate/salicylate screening agents weight ratio will be greater than or equal to 0.5, more preferentially greater than or equal to 0.7.

The non-cyclic volatile silicone oil/mixture of β,β-diphenylacrylate/salicylate screening agents weight ratio may in particular range from 0.5 to 3 and more preferentially from 0.7 to 1.5.

B) Additional oils

The additional oil may be chosen from oils of any type, which are preferably physiologically acceptable oils, especially mineral, animal, plant or synthetic oils; in particular volatile or non-volatile hydrocarbon-based oils and/or silicone oils and/or fluorinated oils, and mixtures thereof.

i) Volatile hydrocarbon-based oils

As examples of volatile hydrocarbon-based oils that may be used in the invention, mention may be made of:

- hydrocarbon-based oils containing from 8 to 16 carbon atoms, and especially $C_8$-$C_{16}$ isoalkanes of petroleum origin (also known as isoparaffins), for instance isododecane (also known as 2,2,4,4,6-pentamethylheptane), isodecane and isohexadecane, for example the oils sold under the trade names Isopar or Permethyl, branched $C_8$-$C_{16}$ esters and isohexyl neopentanoate, and mixtures thereof. Other volatile hydrocarbon-based oils, for instance petroleum distillates, especially those sold under the name Shell Solt by the company Shell, may also be used; volatile linear alkanes, such as those described in patent application DE10 2008 012 457 from the company Cognis.

ii) Non-volatile hydrocarbon-based oils

As examples of non-volatile hydrocarbon-based oils that may be used in the invention, mention may be made of:

- hydrocarbon-based oils of animal origin, such as perhydrosqualene;
- linear or branched hydrocarbons, of mineral or synthetic origin, such as liquid paraffins and derivatives thereof, petroleum jelly, polydecenes, polybutenes, hydrogenated polyisobutene such as Parleam, or squalane;
- phytostearyl esters, such as phytostearyl oleate, phytostearyl isostearate and lauroyl/octyldodecyl/phytostearyl glutamate (Ajinomoto, Eldew PS203®);
- triglycerides constituted of fatty acid esters of glycerol, the fatty acids of which may in particular have chain lengths ranging from $C_4$ to $C_{36}$, and especially from $C_{18}$ to $C_{36}$, these oils possibly being linear or branched, and saturated or unsaturated; these oils may especially be heptanoic or octanoic triglycerides, wheatgerm oil, sunflower oil, grapeseed oil, sesame seed oil (820.6 g/mol), corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, marrow oil, blackcurrant oil, evening primrose oil, millet oil, barley oil, quinoa oil, rye oil, safflower oil, candlenut oil, passionflower oil or musk rose oil; shea oil; or alternatively caprylic/capric acid triglycerides, for instance those sold by the company Stéarineries Dubois or those sold under the names Miglyol 8100, 8120 and 818® by the company Dynamit Nobel;
- synthetic ethers containing from 10 to 40 carbon atoms, such as dicaprylyl ether;
- hydrocarbon-based esters of formula RCOOR’ in which RCOO represents a carboxylic acid residue comprising from 2 to 40 carbon atoms, and R’ represents a hydrocarbon-based chain containing from 1 to 40 carbon atoms, such as cetostearyl octanoate, isopropyl alcohol esters, such as isopropyl myristate or isopropyl palmitate, ethyl palmitate, 2-ethylhexyl palmitate, isopropyl stearate or isostearate, isostearyl isostearate, octyl stearate, diisopropyl adipate, heptanoates, and in particular isostearyl heptanoate, alcohol or polyalcohol octanoates, decanoates or ricinoleates, for instance propylene glycol dioctanoate, cetyl octanoate, tridecyl octanoate, 2-ethylhexyl 4-diheptanoate and palmitate, alkyl benzoate, polyethylene glycol diheptanoate, propylene glycol 2-diethyl hexanoate, and mixtures thereof, C12 to C15 alcohol benzoates, hexyl laurate, neopentanoic acid esters, for instance isodecyl neopentanoate, isotridecyl neopentanoate, isostearyl neopentanoate and 2-octyldodecyl neopentanoate, isononanoic acid esters, for instance isononyl isononanoate, isotridecyl isononanoate and octyl isononanoate, oleyl erucate, isopropyl lauroyl sarcosinate, diisopropyl sebacate, isocetyl stearate, isodecyl neopentanoate, isostearyl behenate, and myristyl myristate;
- polyesters obtained by condensation of unsaturated fatty acid dimer and/or trimer and of diol, such as those described in patent application FR 0 853 634, in particular such as dilinoleic acid and 1,4-butanediol. Mention may in particular be made in this respect of the polymer sold by Biosynthis under the name Viscoplast 14436H® (INCI name: Dilinoleic Acid/Butanediol Copolymer), or copolymers of polyols and of diacid dimers, and esters thereof, such as Hailuscent ISDA®,
- polyol esters and pentaerythritol esters, for instance dipentaerythritol tetrahydroxystearate/tetraisostearate,
- fatty alcohols containing from 12 to 26 carbon atoms, for instance octyldodecanol, 2-butyloctanol, 2-hexyldecanol, 2-undecylpentadecanol and oleyl alcohol;
- dialkyl carbonates, the two alkyl chains possibly being identical or different, such as dicaprylyl carbonate sold under the name Cetiol CC® by Cognis, and
- vinylpyrrolidone copolymers such as the vinylpyrrolidone/1-hexadecene copolymer, Antaron V-216® sold or manufactured by the company ISP,
- linear fatty acid esters with a total carbon number ranging from 35 to 70, such as pentaerythrityl tetrapelargonate,
- hydroxylated esters such as polyglyceryl-2 triisostearate;
- aromatic esters such as tridecyl trimellitate, $C_{12}$-$C_{15}$ alcohol benzoates, the 2-phenylethyl ester of benzoic acid, and butyloctyl salicylate,
- $C_{24}$-$C_{28}$ esters of branched fatty alcohols or fatty acids such as those described in patent application EP-A-0 955 039, and in particular triisoarachidyl citrate, pentaerythrityl tetraisononanoate, glyceryl triisostearate, glyceryl tris(2-decyl)tetradecanoate, pentaerythrityl tetraisostearate, polyglyceryl-2 tetraisostearate or pentaerythrityl tetrakis(2-decyl)tetradecanoate,
- esters and polyesters of dimer diol and of monocarboxylic or dicarboxylic acid, such as esters of dimer diol and of fatty acid and esters of dimer diol and of dimer dicarboxylic acid, such as Lusplan DD-DA5® and Lusplan DD-DA7® sold by the company Nippon Fine Chemical and described in patent application US 2004-175 338.
- and mixtures thereof

According to a particular embodiment, the non-volatile hydrocarbon-based oil is constituted of lipophilic organic UV-screening agents that are liquid at room temperature (25°C) and atmospheric pressure, in particular of the mixture

of β,β-diphenylacrylate/salicylate screening agents as described previously.

Use may also be made of liquid lipophilic cinnamate compounds such as: ethylhexyl methoxycinnamate, sold especially under the trade name Parsol MCX by DSM Nutritional Products,

isopropyl methoxycinnamate,
isoamyl methoxycinnamate, sold under the trade name Neo Heliopan E 1000 by Symrise.

iii) Non-volatile fluoro and/or silicone oils

Among the non-volatile fluoro and/or silicone oils, mention may be made of:

- optionally partially hydrocarbon-based and/or silicone fluoro oils, for instance fluorosilicone oils, fluoropolyethers and fluorosilicones as described in document EP-A-847 752,
- silicone oils, such as non-volatile polydimethylsiloxanes (PDMSs); phenyl silicones, such as phenyl trimethicones, phenyl dimethicones, phenyl(trimethylsiloxy)diphenylsiloxanes, diphenyl dimethicones and diphenyl(methyldiphenyl)trisiloxanes.

iv) Volatile cyclic silicone oils

As volatile cyclic silicone oil, mention may be made of octamethylcyclotetrasiloxane, decamethylcyclopentasiloxane and dodecamethylcyclohexasiloxane, and mixtures thereof.

**PIGMENTS**

The composition according to the invention comprises at least one pigment.

The term "pigments" means white or coloured, mineral or organic particles, which are insoluble in an aqueous medium, and which are intended to colour and/or opacify the resulting composition and/or deposit. These pigments may be white or coloured, and mineral and/or organic.

Preferably, the composition comprises at least 5% by weight of pigments, more preferentially from 5% to 40% by weight of pigments, in particular from 10% to 30% by weight and preferably from 10% to 20% by weight of pigments, relative to the total weight of said composition.

According to a particular embodiment, the pigments used according to the invention are chosen from mineral pigments.

The term "mineral pigment" means any pigment that satisfies the definition in Ullmann's encyclopaedia in the chapter on inorganic pigments. Among the mineral pigments that are useful in the present invention, mention may be made of zirconium oxide or cerium oxide, and also zinc oxide, iron oxide (black, yellow or red) or chromium oxide, manganese violet, ultramarine blue, chromium hydrate and ferric blue, titanium dioxide, and metal powders, for instance aluminium powder or copper powder. The following mineral pigments may also be used: $Ta_2O_5$, TisOs, $Ti_2O_3$, TiO, $ZrO_2$ as a mixture with $TiO_2$, $ZrO_2$, $Nb_2O_5$, $CeO_2$, ZnS.

The size of the pigment that is useful in the context of the present invention is generally greater than 100 nm and may range up to 10 μm, preferably from 200 nm to 5 μm and more preferentially from 300 nm to 1 μm.

According to a particular form of the invention, the pigments have a size characterized by a D[50] greater than 100 nm and possibly ranging up to 10 μm, preferably from 200 nm to 5 μm and more preferentially from 300 nm to 1 μm.

The sizes are measured by static light scattering using a commercial MasterSizer 3000 particle size analyser from Malvern, which makes it possible to determine the particle size distribution of all of the particles over a wide range which may extend from 0.01 μm to 1000 μm. The data are processed on the basis of the standard Mie scattering theory. This theory is the most suitable for size distributions ranging from submicron to multimicron; it allows an "effective" particle diameter to be determined. This theory is especially described in the publication by Van de Hulst, H.C., Light Scattering by Small Particles, Chapters 9 and 10, Wiley, New York, 1957.

D[50] represents the maximum size that 50% by volume of the particles have.

In the context of the present invention, the mineral pigments are more particularly iron oxide and/or titanium dioxide. Examples that may be mentioned more particularly include titanium dioxide and iron oxide coated with aluminium stearoyl glutamate, sold, for example, under the reference NAI by the company Miyoshi Kasei.

As mineral pigments that may be used in the invention, mention may also be made of nacres.

The term "nacres" should be understood as meaning coloured particles of any form, which may or may not be iridescent, especially produced by certain molluscs in their shell, or alternatively synthesized, and which have a colour effect via optical interference.

The nacres may be chosen from nacreous pigments such as titanium mica coated with an iron oxide, titanium

mica coated with bismuth oxychloride, titanium mica coated with chromium oxide, titanium mica coated with an organic dye and also nacreous pigments based on bismuth oxychloride. They may also be mica particles, at the surface of which are superposed at least two successive layers of metal oxides and/or of organic dyestuffs.

Examples of nacres that may also be mentioned include natural mica covered with titanium oxide, with iron oxide, with natural pigment or with bismuth oxychloride. Among the nacres available on the market, mention may be made of the nacres Timica, Flamenco and Duochrome (based on mica) sold by the company Engelhard, the Timiron nacres sold by the company Merck, the Prestige mica-based nacres sold by the company Eckart, and the Sunshine synthetic mica-based nacres sold by the company Sun Chemical.

The nacres may more particularly have a yellow, pink, red, bronze, orangey, brown, gold and/or coppery colour or glint.

As illustrations of nacres that may be used in the context of the present invention, mention may be made in particular of gold-coloured nacres sold especially by the company Engelhard under the name Brilliant gold 212G (Timica), Gold 222C (Cloisonne), Sparkle gold (Timica), Gold 4504 (Chromalite) and Monarch gold 233X (Cloisonne); the bronze nacres sold especially by the company Merck under the names Bronze fine (17384) (Colorona) and Bronze (17353) (Colorona) and by the company Engelhard under the name Super bronze (Cloisonne); the orange nacres sold especially by the company Engelhard under the names Orange 363C (Cloisonne) and Orange MCR 101 (Cosmica) and by the company Merck under the names Passion orange (Colorona) and Matte orange (17449) (Microna); the brown-tinted nacres sold especially by the company Engelhard under the names Nuantique copper 340XB (Cloisonne) and Brown CL4509 (Chromalite); the nacres with a copper glint sold especially by the company Engelhard under the name Copper 340A (Timica); the nacres with a red glint sold especially by the company Merck under the name Sienna fine (17386) (Colorona); the nacres with a yellow glint sold especially by the company Engelhard under the name Yellow (4502) (Chromalite); the red-tinted nacres with a golden glint sold especially by the company Engelhard under the name Sunstone G012 (Gemtone); the pink nacres sold especially by the company Engelhard under the name Tan opal G005 (Gemtone); the black nacres with a golden glint sold especially by the company Engelhard under the name Nu antique bronze 240 AB (Timica); the blue nacres sold especially by the company Merck under the name Matte blue (17433) (Microna); the white nacres with a silvery glint sold especially by the company Merck under the name Xirona Silver; and the golden-green pinkish-orange nacres sold especially by the company Merck under the name Indian summer (Xirona), and mixtures thereof.

Among the pigments that may be used according to the invention, mention may also be made of those having an optical effect different from a simple conventional colouring effect, that is to say a unified and stabilized effect such as produced by conventional dyestuffs, for instance monochromatic pigments. For the purposes of the invention, the term "stabilized" means lacking an effect of variability of the colour as a function of the angle of observation or alternatively in response to a temperature change.

For example, this material may be chosen from particles with a metallic glint, goniochromatic colouring agents, diffracting pigments, thermochromic agents, optical brighteners, and also fibres, especially interference fibres. Needless to say, these various materials may be combined in order simultaneously to afford two effects, or even a novel effect in accordance with the invention.

The particles with a metallic glint that may be used in the invention are in particular chosen from:

- particles of at least one metal and/or of at least one metal derivative,
- particles comprising a monomaterial or multimaterial organic or mineral substrate, at least partially coated with at least one layer with a metallic glint comprising at least one metal and/or at least one metal derivative, and

mixtures of said particles.

Among the metals that may be present in said particles, mention may be made, for example, of Ag, Au, Cu, Al, Ni, Sn, Mg, Cr, Mo, Ti, Zr, Pt, Va, Rb, W, Zn, Ge, Te and Se, and mixtures or alloys thereof. Ag, Au, Cu, Al, Zn, Ni, Mo and Cr, and mixtures or alloys thereof (for example bronzes and brasses) are preferred metals.

The term "metal derivatives" denotes compounds derived from metals, especially oxides, fluorides, chlorides and sulfides.

Illustrations of these particles that may be mentioned include aluminium particles, such as those sold under the names Starbrite 1200 EAC® by the company Silberline and Metalure® by the company Eckart.

Mention may also be made of metal powders of copper or of alloy mixtures such as the references 2844 sold by the company Radium Bronze, metallic pigments, for instance aluminium or bronze, such as those sold under the names Rotosafe 700 from the company Eckart, silica-coated aluminium particles sold under the name Visionaire Bright Silver from the company Eckart, and metal alloy particles, for instance the silica-coated bronze (alloy of copper and zinc) powders sold under the name Visionaire Bright Natural Gold from the company Eckart.

They may also be particles comprising a glass substrate, such as those sold by the company Nippon Sheet Glass under the name Microglass Metashine.

The goniochromatic colouring agent may be chosen, for example, from interference multilayer structures and liquid-crystal colouring agents.

Examples of symmetrical multilayer interference structures that may be used in the compositions prepared in accordance with the invention are, for example, the following structures: $Al/SiO_2/Al/SiO_2/Al$, pigments having this structure being sold by the company DuPont de Nemours; $Cr/MgF_2/Al/MgF_2/Cr$, pigments having this structure being sold under the name Chromaflair by the company Flex; $MoS_2/SiO_2/Al/SiO_2/MoS_2$; $Fe_2O_3/SiO_2/Al/SiO_2/Fe_2O_3$, and $Fe_2O_3/SiO_2/Fe_2O_3/SiO_2/Fe_2O_3$, pigments having these structures being sold under the name Sicopearl by the company BASF; $MoS_2/SiO_2$/mica-oxide/$SiO_2/MoS_2$; $Fe_2O_3/SiO_2$/mica-oxide/$SiO_2/Fe_2O_3$; $TiO_2/SiO_2/TiO_2$ and $TiO_2/Al_2O_3/TiO_2$; $SnO/TiO_2/SiO_2/TiO_2/SnO$; $Fe_2O_3/SiO_2/Fe_2O_3$; SnO/mica/$TiO_2/SiO_2/TiO_2$/mica/SnO, pigments having these structures being sold under the name Xirona by the company Merck (Darmstadt). By way of example, these pigments may be the pigments of silica/titanium oxide/tin oxide structure sold under the name Xirona Magic by the company Merck, the pigments of silica/brown iron oxide structure sold under the name Xirona Indian Summer by the company Merck and the pigments of silica/titanium oxide/mica/tin oxide structure sold under the name Xirona Caribbean Blue by the company Merck. Mention may also be made of the Infinite Colors pigments from the company Shiseido. Depending on the thickness and the nature of the various coats, different effects are obtained. Thus, with the structure $Fe_2O_3/SiO_2/Al/SiO_2/Fe_2O_3$, the colour changes from green-golden to red-grey for $SiO_2$ layers of 320 to 350 nm; from red to golden for $SiO_2$ layers of 380 to 400 nm; from purple to green for $SiO_2$ layers of 410 to 420 nm; and from copper to red for $SiO_2$ layers of 430 to 440 nm.

As examples of pigments with a polymeric multilayer structure, mention may be made of those sold by the company 3M under the name Color Glitter.

Examples of liquid-crystal goniochromatic particles that may be used include those sold by the company Chenix and also the product sold under the name Helicone® HC by the company Wacker.

Hydrophobic coated pigments

Preferably, the compositions according to the invention comprise at least one pigment coated with at least one lipophilic or hydrophobic compound and especially as detailed below.

This type of pigment is particularly advantageous insofar as it may be considered in large amount together with a large amount of water. What is more, insofar as they are treated with a hydrophobic compound, they show a predominant affinity for the oily gelled phase, which can then convey them.

Needless to say, the compositions according to the invention may in parallel contain uncoated pigments.

The coating may also comprise at least one additional non-lipophilic compound. For the purposes of the invention, the "coating" of a pigment according to the invention generally denotes the total or partial surface treatment of the pigment with a surface agent, absorbed, adsorbed or grafted onto said pigment.

The surface-treated pigments may be prepared according to surface treatment techniques of chemical, electronic, mechanochemical or mechanical nature that are well known to those skilled in the art. Commercial products may also be used.

The surface agent may be absorbed, adsorbed or grafted onto the pigments by evaporation of solvent, chemical reaction and creation of a covalent bond.

According to one variant, the surface treatment consists of coating of the pigments.

The coating may represent from 0.1% to 20% by weight and in particular from 0.5% to 5% by weight, relative to the total weight of the coated pigment.

The coating may be performed, for example, by adsorption of a liquid surface agent onto the surface of the solid particles by simple mixing with stirring of the particles and of said surface agent, optionally with heating, prior to the incorporation of the particles into the other ingredients of the makeup or care composition.

The coating may be performed, for example, by chemical reaction of a surface agent with the surface of the solid pigment particles and creation of a covalent bond between the surface agent and the particles. This method is especially described in patent US 4 578 266.

The chemical surface treatment may consist in diluting the surface agent in a volatile solvent, dispersing the pigments in this mixture and then slowly evaporating off the volatile solvent, so that the surface agent is deposited at the surface of the pigments.

Lipophilic or hydrophobic treatment agent

When the pigment comprises a lipophilic or hydrophobic coating, it is preferably present in the fatty phase of the composition according to the invention.

According to a particular embodiment of the invention, the pigments may be coated according to the invention with at least one compound chosen from silicone surface agents; fluoro surface agents; fluorosilicone surface agents;

metal soaps; N-acylamino acids or salts thereof; lecithin and derivatives thereof; isopropyl triisostearyl titanate; isostearyl sebacate; natural plant or animal waxes; polar synthetic waxes; fatty esters; phospholipids; and mixtures thereof.

Silicone surface agent

According to a particular embodiment, the pigments may be totally or partially surface-treated with a compound of silicone nature.

The silicone surface agents may be chosen from organopolysiloxanes, silane derivatives, silicone-acrylate copolymers, silicone resins, and mixtures thereof.

The term "organopolysiloxane compound" means a compound having a structure comprising an alternance of silicon atoms and oxygen atoms and comprising organic radicals linked to silicon atoms.

i) Non-elastomeric organopolysiloxane

Non-elastomeric organopolysiloxanes that may especially be mentioned include polydimethylsiloxanes, polymethylhydrogenosiloxanes and polyalkoxydimethylsiloxanes.

The alkoxy group may be represented by the radical R-O- such that R represents methyl, ethyl, propyl, butyl or octyl, 2-phenylethyl, 2-phenylpropyl or 3,3,3-trifluoropropyl radicals, aryl radicals such as phenyl, tolyl or xylyl, or substituted aryl radicals such as phenylethyl.

One method for surface-treating pigments with a polymethylhydrogenosiloxane consists in dispersing the pigments in an organic solvent and then in adding the silicone compound. On heating the mixture, covalent bonds are created between the silicone compound and the surface of the pigment.

According to a preferred embodiment, the silicone surface agent may be a non-elastomeric organopolysiloxane, especially chosen from polydimethylsiloxanes.

ii) Alkylsilanes and alkoxysilanes

Silanes bearing alkoxy functionality are especially described by Witucki in "A silane primer, Chemistry and applications of alkoxy silanes, Journal of Coatings Technology, 65, 822, pages 57-60, 1993".

Alkoxysilanes such as the alkyltriethoxysilanes and the alkyltrimethoxysilanes sold under the references Silquest A-137 (OSI Specialities) and Prosil 9202 (PCR) may be used for coating the pigments.

The use of alkylpolysiloxanes bearing a reactive end group such as alkoxy, hydroxyl, halogen, amino or imino is described in patent application JP H07-196946. They are also suitable for treating the pigments.

iii) Silicone-acrylate polymers

Grafted silicone-acrylic polymers having a silicone backbone as described in patents US 5 725 882, US 5 209 924, US 4 972 037, US 4 981 903, US 4 981 902 and US 5 468 477 and in patents US 5 219 560 and EP 0 388 582 may be used.

Other silicone-acrylate polymers may be silicone polymers comprising in their structure the unit of formula (I) below:

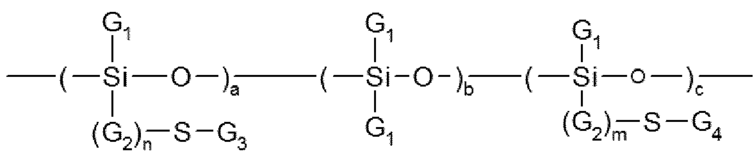

(I)

in which the radicals $G_1$, which may be identical or different, represent hydrogen or a $C_1$-$C_{10}$ alkyl radical or alternatively a phenyl radical; the radicals $G_2$, which may be identical or different, represent a $C_1$-$C_{10}$ alkylene group; $G_3$ represents a polymeric residue resulting from the (homo)polymerization of at least one ethylenically unsaturated anionic monomer; $G_4$ represents a polymeric residue resulting from the (homo)polymerization of at least one ethylenically unsaturated hydrophobic monomer; m and n are equal to 0 or 1; a is an integer ranging from 0 to 50; b is an integer that may be between 10 and 350, c is an integer ranging from 0 to 50; with the proviso that one of the parameters a and c is other than 0.

Preferably, the unit of formula (I) above has at least one, and even more preferentially all, of the following characteristics:

- the radicals G1 denote an alkyl radical, preferably a methyl radical;
- n is non-zero, and the radicals G2 represent a divalent C1-C3 radical, preferably a propylene radical;
- G3 represents a polymeric radical resulting from the (homo)polymerization of at least one monomer of the ethylenically unsaturated carboxylic acid type, preferably acrylic acid and/or methacrylic acid;
- G4 represents a polymeric radical resulting from the (homo)polymerization of at least one monomer of the (C1-C10)alkyl (meth)acrylate type, preferably such as isobutyl or methyl (meth)acrylate.

Examples of silicone polymers corresponding to formula (I) are especially polydimethylsiloxanes (PDMS) onto which are grafted, via a connecting chain unit of thiopropylene type, mixed polymer units of the poly(meth)acrylic acid type and of the polymethyl (meth)acrylate type.
Other examples of silicone polymers corresponding to formula (I) are especially polydimethylsiloxanes (PDMS) onto which are grafted, via a connecting chain unit of thiopropylene type, polymer units of the polyisobutyl (meth)acrylate type.

iv) Silicone resins

The silicone surface agent may be chosen from silicone resins.
The term "resin" means a three-dimensional structure.
The silicone resins may be soluble or swellable in silicone oils. These resins are crosslinked polyorganosiloxane polymers.
The nomenclature of silicone resins is known under the name "MDTQ", the resin being described as a function of the various siloxane monomer units that it comprises, each of the letters "MDTQ" characterizing a type of unit.
The letter M represents the monofunctional unit of formula $(CH_3)_3SiO_{1/2}$, the silicon atom being connected to only one oxygen atom in the polymer comprising this unit.
The letter D means a difunctional unit $(CH_3)_2SiO_{2/2}$ in which the silicon atom is bonded to two oxygen atoms.
The letter T represents a trifunctional unit of formula $(CH_3)SiO_{3/2}$.
In the units M, D and T defined previously, at least one of the methyl groups may be substituted with a group R other than a methyl group, such as a hydrocarbon-based radical (especially alkyl) containing from 2 to 10 carbon atoms or a phenyl group, or alternatively a hydroxyl group.
Finally, the letter Q means a tetrafunctional unit $SiO_{4/2}$ in which the silicon atom is bonded to four hydrogen atoms, which are themselves bonded to the rest of the polymer.
Various resins with different properties may be obtained from these different units, the properties of these polymers varying as a function of the type of monomers (or units), of the type and number of substituted radicals, of the length of the polymer chain, of the degree of branching and of the size of the side chains.
Examples of these silicone resins that may be mentioned include:

- siloxysilicates, which may be trimethyl siloxysilicates of formula $[(CH_3)_3XSiXO]_xX(SiO_{4/2})_y$ (MQ units) in which x and y are integers ranging from 50 to 80;
- polysilsesquioxanes of formula $(CH_3SiO_{3/2})_x$ (T units) in which x is greater than 100 and at least one of the methyl radicals of which may be substituted with a group R as defined above;
- polymethylsilsesquioxanes, which are polysilsesquioxanes in which none of the methyl radicals is substituted with another group.

Such polymethylsilsesquioxanes are described in US 5 246 694.
As examples of commercially available polymethylsilsesquioxane resins, mention may be made of those sold:

- by the company Wacker under the reference Resin MK, such as Belsil PMS MK: polymer comprising $CH_3SiO_{3/2}$ repeating units (T units), which may also comprise up to 1% by weight of $(CH_3)_2SiO_{2/2}$ units (D units) and having an average molecular weight of about 10 000;
- by the company Shin-Etsu under the references KR-220L, which are composed of units T of formula $CH_3SiO_{3/2}$ and contain Si-OH (silanol) end groups, under the reference KR-242A, which comprise 98% of units T and 2% of dimethyl units D and contain Si-OH end groups, or else under the reference KR-251, comprising 88% of units T and 12% of dimethyl units D and contain Si-OH end groups.

Siloxysilicate resins that may be mentioned include trimethylsiloxysilicate (TMS) resins, optionally in the form of powders. Such resins are sold under the references SR1000, E 1 170-002 or SS 4230, by the company General Electric or under the references TMS 803, Wacker 803 and 804 by the company Wacker Silicone Corporation.
Mention may also be made of trimethylsiloxysilicate resins sold in a solvent such as cyclomethicone, sold under

the name KF-7312J by the company Shin-Etsu or DC 749 and DC 593 by the company Dow Corning.

As examples of commercial references of pigments treated with a silicone compound, mention may be made of:

- red iron oxide/dimethicone sold under the reference SA-C 338075-10 by the company Miyoshi Kasei, and
- a pigment obtained by treating DC Red 7 with a silicone compound, sold by the company Coletica under the reference Gransil GCM (which is a mixture of D5 and polysilicone 11).

Fluoro surface agent

The pigments may be totally or partially surface-treated with a compound of fluoro nature.

The fluoro surface agents may be chosen from perfluoroalkyl phosphates, perfluoropolyethers, polytetrafluoropolyethylenes (PTFE), perfluoroalkanes, perfluoroalkyl silazanes, polyhexafluoropropylene oxides, and polyorganosiloxanes comprising perfluoroalkyl perfluoropolyether groups.

The term "perfluoroalkyl radical" means an alkyl radical in which all the hydrogen atoms have been replaced with fluorine atoms.

Perfluoropolyethers are especially described in patent application EP 0 486 135, and sold under the trade name Fomblin by the company Montefluos.

Perfluoroalkyl phosphates are in particular described in patent application JP H05-86984. The perfluoroalkyl diethanolamine phosphates sold by Asahi Glass under the reference AsahiGuard AG530 may be used.

Among the linear perfluoroalkanes that may be mentioned are perfluorocycloalkanes, perfluoro(alkylcycloalkanes), perfluoropolycycloalkanes, aromatic perfluoro hydrocarbons (perfluoroarenes) and hydrocarbon-based perfluoro organic compounds comprising at least one heteroatom.

Among the perfluoroalkanes, mention may be made of the linear alkane series such as perfluorooctane, perfluorononane or perfluorodecane.

Among the perfluorocycloalkanes and perfluoro(alkylcycloalkanes), mention may be made of perfluorodecalin sold under the name Flutec PP5 GMP by the company Rhodia, perfluoro(methyldecalin) and perfluoro(C3-C5 alkylcyclohexanes) such as perfluoro(butylcyclohexane).

Among the perfluoropolycycloalkanes, mention may be made of bicyclo[3.3.1]nonane derivatives such as perfluorotrimethylbicyclo[3.3.1]nonane, adamantane derivatives such as perfluorodimethyladamantane, and hydrogenated perfluorophenanthrene derivatives such as tetracosafluorotetradecahydrophenanthrene.

Among the perfluoroarenes, mention may be made of perfluoronaphthalene derivatives, for instance perfluoronaphthalene and perfluoromethyl-1-naphthalene.

As examples of commercial references of pigments treated with a fluoro compound, mention may be made of:

- yellow iron oxide/perfluoroalkyl phosphate sold under the reference PF 5 Yellow 601 by the company Daito Kasei;
- red iron oxide/perfluoroalkyl phosphate sold under the reference PF 5 Red R 516L by the company Daito Kasei;
- black iron oxide/perfluoroalkyl phosphate sold under the reference PF 5 Black BL 100 by the company Daito Kasei;
- titanium dioxide/perfluoroalkyl phosphate sold under the reference PF 5 TiO2 CR 50 by the company Daito Kasei;
- yellow iron oxide/perfluoropolymethyl isopropyl ether sold under the reference Iron oxide yellow BF-25-3 by the company Toshiki;
- DC Red 7/perfluoropolymethyl isopropyl ether sold under the reference D&C Red 7 FHC by the company Cardre Inc.; and
- DC Red 6/PTFE sold under the reference T 9506 by the company Warner-Jenkinson.

Fluorosilicone surface agent

The pigments may be totally or partially surface-treated with a compound of fluorosilicone nature.

The fluorosilicone compound may be chosen from perfluoroalkyl dimethicones, perfluoroalkyl silanes and perfluoroalkyl trialkoxysilanes.

Perfluoroalkyl silanes that may be mentioned include the products LP-IT and LP-4T sold by Shin-Etsu Silicone.

The perfluoroalkyl dimethicones may be represented by the following formula:

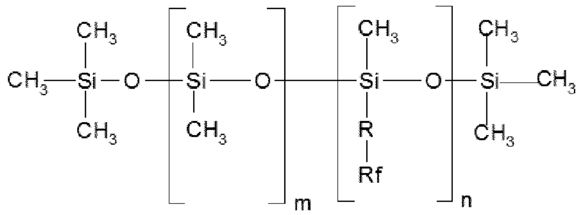

in which:

- R represents a linear or branched divalent alkyl group containing from 1 to 6 carbon atoms, preferably a divalent methyl, ethyl, propyl or butyl group;
- Rf represents a perfluoroalkyl radical containing 1 to 9 carbon atoms and preferably 1 to 4 carbon atoms;
- m is chosen between 0 and 150 and preferably from 20 to 100; and
- n is chosen between 1 and 300 and preferably between 1 and 100.

As examples of commercial references of pigments treated with a fluorosilicone compound, mention may be made of titanium dioxide/fluorosilicone sold under the reference Fluorosil Titanium dioxide 100TA by the company Advanced Dermaceuticals International Inc.

<u>Other lipophilic surface agents</u>

The hydrophobic treatment agent may also be chosen from:

i) metal soaps such as aluminium dimyristate and the aluminium salt of hydrogenated tallow glutamate;
Metal soaps that may especially be mentioned include metal soaps of fatty acids containing from 12 to 22 carbon atoms and in particular those containing from 12 to 18 carbon atoms.

The metal of the metal soap may especially be zinc or magnesium.
Metal soaps that may be used include zinc laurate, magnesium stearate, magnesium myristate and zinc stearate, and mixtures thereof.

ii) fatty acids such as lauric acid, myristic acid, stearic acid and palmitic acid;
iii) N-acylamino acids or salts thereof, which may comprise an acyl group containing from 8 to 22 carbon atoms, for instance a 2-ethylhexanoyl, caproyl, lauroyl, myristoyl, palmitoyl, stearoyl or cocoyl group;

The amino acid may be, for example, lysine, glutamic acid or alanine.
The salts of these compounds can be the aluminium, magnesium, calcium, zirconium, zinc, sodium or potassium salts.
Thus, according to a particularly preferred embodiment, an N-acylamino acid derivative may in particular be a glutamic acid derivative and/or a salt thereof, and more particularly a stearoyl glutamate, for instance aluminium stearoyl glutamate.

iv) lecithin and derivatives thereof;
v) isopropyl triisostearyl titanate;

As examples of isopropyl titanium triisostearate (ITT)-treated pigments, mention may be made of those sold under the commercial references BWBO-I2 (Iron oxide CI77499 and isopropyl titanium triisostearate), BWYO-I2 (Iron oxide CI77492 and isopropyl titanium triisostearate) and BWRO-I2 (Iron oxide CI77491 and isopropyl titanium triisostearate) by the company Kobo.

vi) isostearyl sebacate;
vii) natural plant or animal waxes or polar synthetic waxes;
viii) fatty esters, in particular jojoba esters;
ix) phospholipids; and
x) mixtures thereof.

The waxes mentioned in the compounds mentioned previously may be those generally used in cosmetics, as

defined hereinbelow.

They may especially be hydrocarbon-based, silicone and/or fluoro waxes, optionally comprising ester or hydroxyl functions. They may also be of natural or synthetic origin.

The term "polar wax" means a wax containing chemical compounds comprising at least one polar group. Polar groups are well known to those skilled in the art; they may be, for example, alcohol, ester or carboxylic acid groups. Polyethylene waxes, paraffin waxes, microcrystalline waxes, ozokerite and Fischer-Tropsch waxes are not included among polar waxes.

In particular, the polar waxes have a mean Hansen solubility parameter δa at 25°C such that δa > 0 $(J/cm^3)^{1/2}$ and better still δa > 1 $(J/cm^3)^{1/2}$:

$$\delta_a = \sqrt{\delta_p^2 + \delta_h^2}$$

in which δp and δh are, respectively, the polar contributions and contributions of interaction types specific to the Hansen solubility parameters.

The definition of solvents in the three-dimensional solubility space according to Hansen is described in the article by C.M. Hansen: "The three-dimensional solubility parameters", J. Paint Technol. 39, 105 (1967):

- δh characterizes the specific interaction forces (such as hydrogen bonding, acid/base, donor/acceptor, etc.);
- δp characterizes the Debye interaction forces between permanent dipoles and also the Keesom interaction forces between induced dipoles and permanent dipoles.

The parameters δp and δh are expressed in $(J/cm^3)^{1/2}$.

A polar wax is especially formed from molecules comprising, besides carbon and hydrogen atoms in their chemical structure, heteroatoms (such as O, N and P).

Non-limiting illustrations of these polar waxes that may especially be mentioned include natural polar waxes, such as beeswax, lanolin wax, orange wax, lemon wax and Chinese insect waxes, rice bran wax, carnauba wax, candelilla wax, ouricury wax, cork fibre wax, sugarcane wax, Japan wax, sumac wax and montan wax.

According to a particular embodiment, the pigments may be coated with at least one compound chosen from silicone surface agents; fluoro surface agents; N-acylamino acids or salts thereof; isopropyl triisostearyl titanate; natural plant or animal waxes; fatty esters; and mixtures thereof.

According to a particularly preferred embodiment, the pigments may be coated with an N-acylamino acid and/or a salt thereof, in particular with a glutamic acid derivative and/or a salt thereof, or with a fatty ester, in particular with a jojoba ester.

According to a more particularly preferred embodiment, the pigments may be coated with an N-acylamino acid and/or a salt thereof, in particular with a glutamic acid derivative and/or a salt thereof, especially a stearoyl glutamate, for instance aluminium stearoyl glutamate.

Examples of coated pigments according to the invention that may be mentioned more particularly include titanium dioxide and iron oxide coated with aluminium stearoyl glutamate, sold, for example, under the reference NAI by Miyoshi Kasei.

Pigments not coated with a hydrophobic compound

As stated previously, a composition may also contain pigments not coated with a lipophilic or hydrophobic compound.

These other pigments may be coated with a hydrophilic compound or uncoated.

These pigments may be mineral pigments especially as defined previously.

These pigments may also be organic pigments.

The term "organic pigment" means any pigment that satisfies the definition in Ullmann's encyclopaedia in the chapter on organic pigments. The organic pigment may in particular be chosen from nitroso, nitro, azo, xanthene, quinoline, anthraquinone, phthalocyanin, metal complex type, isoindolinone, isoindoline, quinacridone, perinone, perylene, diketopyrrolopyrrole, thioindigo, dioxazine, triphenylmethane and quinophthalone compounds.

The organic pigment(s) may be chosen, for example, from carmine, carbon black, aniline black, melanin, azo yellow, quinacridone, phthalocyanin blue, sorghum red, the blue pigments codified in the Color Index under the references CI 42090, 69800, 69825, 73000, 74100 and 74160, the yellow pigments codified in the Color Index under the references CI 11680, 11710, 15985, 19140, 20040, 21100, 21108, 47000 and 47005, the green pigments codified in the Color Index under the references CI 61565, 61570 and 74260, the orange pigments codified in the Color Index under the references

CI 11725, 15510, 45370 and 71105, the red pigments codified in the Color Index under the references CI 12085, 12120, 12370, 12420, 12490, 14700, 15525, 15580, 15620, 15630, 15800, 15850, 15865, 15880, 17200, 26100, 45380, 45410, 58000, 73360, 73915 and 75470, and the pigments obtained by oxidative polymerization of indolic or phenolic derivatives as described in patent FR 2 679 771.

These pigments may also be in the form of composite pigments as described in patent EP 1 184 426. These composite pigments may especially be composed of particles comprising a mineral core at least partially covered with an organic pigment and at least one binder for fixing the organic pigments to the core.

The pigment may also be a lake. The term "lake" means insolubilized dyes adsorbed onto insoluble particles, the assembly thus obtained remaining insoluble during use.

The mineral substrates onto which the dyes are adsorbed are, for example, alumina, silica, calcium sodium borosilicate or calcium aluminium borosilicate and aluminium.

Among the organic dyes, mention may be made of cochineal carmine. Mention may also be made of the products known under the following names: D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green (CI 61 570), D&C Yellow 1 O (CI 77 002), D&C Green 3 (CI 42 053), D&C Blue 1 (CI 42 090).

An example of a lake that may be mentioned is the product known under the name D&C Red 7 (CI 15 850:1).

Nature of the hydrophilic coating

As stated previously, these other pigments may be coated with a hydrophilic compound.

Said hydrophilic compound for surface-treating a pigment in order to optimize its dispersion in the gelled aqueous phase is more particularly chosen from biological polymers, carbohydrates, polysaccharides, polyacrylates and polyethylene glycol derivatives.

As examples of biological polymers, mention may be made of polymers based on monomers of carbohydrate type.

More particularly, mention may be made of biosaccharide gum; chitosans and derivatives thereof, such as butoxy chitosan, carboxymethyl chitosan, carboxybutyl chitosan, chitosan gluconate, chitosan adipate, chitosan glycolate, chitosan lactate, etc.; chitins and derivatives thereof, such as carboxymethyl chitin, chitin glycolate; cellulose and derivatives thereof such as cellulose acetate; microcrystalline cellulose; distarch phosphate; sodium hyaluronate; soluble proteoglycans; galacto-arabinans; glycosaminoglycans; glycogen; sclerotium gum; dextran; starch and derivatives thereof; and mixtures thereof.

Examples of carbohydrates that may especially be mentioned include polyhydroxyaldehydes and polyhydroxy ketones of general formula: $C_x(H_2O)_y$ in which x and y may range from 1 to 1 000 000.

The carbohydrates may be monosaccharides, disaccharides or polysaccharides.

Examples of carbohydrates that may especially be mentioned include amylodextrins, beta-glucans, cyclodextrins, modified corn starch, glycogen, hyaluronic acid, hydroxypropylcyclodextrin, lactose, maltitol, guanosine, glyceryl starch, *Triticum vulgare* starch, trehalose, sucrose and derivatives thereof, raffinose and sodium chondroitin sulfate.

$C_1$-$C_{20}$ alkylene glycols or $C_1$-$C_{20}$ alkylene glycol ethers, alone or used in combination with tri($C_1$-$C_{20}$)alkylsilanes, may also be used as surface-treatment agents.

Examples that may be mentioned include pigments surface-treated with PEG alkyl ether alkoxysilane, for instance pigments treated with PEG-8-methyl ether triethoxysilane sold by the company Kobo under the name SW pigments.

Silicones such as dimethicones bearing hydrophilic groups, also known under the name dimethicone copolyols or alkyl dimethicone copolyols, may also be suitable for use in the invention as surface treatment agents. In particular, such dimethicones may comprise, as repeating units, $C_1$-$C_{20}$ alkylene oxides, such as ethylene or propylene oxides.

An example that may be mentioned is the pigment treated with PEG-12-dimethicone, sold by the company Sensient Corporation under the name LCW AQ Pigment.

The amount of pigments coated with at least one hydrophilic compound and/or of uncoated pigments is especially conditioned by the intended use of the cosmetic composition under consideration, and the adjustment of this amount obviously falls within the competence of the composition formulator.

## PRESENTATION FORMS

The compositions according to the invention comprise an oily continuous phase.

According to a particular form of the invention, they may be in the form of an anhydrous composition such as an oil, a cream, a balm or an oily gel (oleogel).

For the purposes of the present invention, the term "anhydrous" refers to a composition comprising a content

of less than or equal to 2% by weight, preferably less than or equal to 1% and more preferentially less than 0.5% by weight of water relative to the total weight of said composition, or is even free of water. Where appropriate, such small amounts of water may especially be introduced by ingredients of the composition that may contain residual amounts thereof.

According to a particularly preferred form, the compositions are in the form of a water-in-oil emulsion comprising an oily continuous phase and an aqueous phase dispersed in said oily phase. They may be in the form of a more or less fluid or thick cream.

According to a particular form of the invention, the composition is a water-in-oil emulsion comprising at least:

a) an oily continuous phase;
b) an aqueous discontinuous phase dispersed in said oily phase;
c) a mixture of organic UV-screening agents comprising i) Octocrylene and ii) ethylhexyl salicylate and/or Homosalate; and
d) at least one non-cyclic volatile silicone oil, chosen in particular from octamethyltrisiloxane, decamethyltetrasiloxane and dodecamethylpentasiloxane, and mixtures thereof, and more particularly dodecamethylpentasiloxane.

According to a particular form of the invention, the composition is a water-in-oil emulsion comprising at least:

a) an oily continuous phase;
b) an aqueous discontinuous phase dispersed in said oily phase;
c) at least 15% by weight, relative to the total weight of the composition, of a mixture of organic UV-screening agents comprising i) Octocrylene and ii) ethylhexyl salicylate and/or Homosalate; and
d) at least one non-cyclic volatile silicone oil, chosen in particular from octamethyltrisiloxane, decamethyltetrasiloxane and dodecamethylpentasiloxane, and mixtures thereof, and more particularly dodecamethylpentasiloxane; the weight ratio of said non-cyclic volatile silicone oil to said mixture of organic UV-screening agents being at least 0.5 and more particularly at least 0.7.

**A) Aqueous phase**

The aqueous phase comprises water and optionally water-soluble or water-miscible ingredients, such as water-soluble solvents.

A water that is suitable for use in the invention may be a floral water such as cornflower water and/or a mineral water such as Vittel water, Lucas water or La Roche Posay water and/or a thermal spring water.

In the present invention, the term "water-soluble solvent" denotes a compound that is liquid at room temperature and water-miscible (miscibility with water of greater than 50% by weight at 25°C and atmospheric pressure).

The water-soluble solvents that may be used in the composition of the invention may also be volatile.

Among the water-soluble solvents that may be used in the composition in accordance with the invention, mention may be made especially of lower monoalcohols containing from 1 to 5 carbon atoms such as ethanol and isopropanol, glycols containing from 2 to 8 carbon atoms such as ethylene glycol, propylene glycol, 1,3-butylene glycol and dipropylene glycol, $C_3$ and $C_4$ ketones and $C_2$-$C_4$ aldehydes.

When the composition is a water-in-oil emulsion, the aqueous phase is preferably present in a concentration of at least 20% by weight, preferably ranging from 30% to 60% by weight, more particularly from 40% to 50% by weight, relative to the total weight of said composition.

**B) Emulsifiers**

The water-in-oil emulsions according to the invention generally comprise one or more emulsifying surfactants, which are preferably nonionic.

For the purposes of the present invention, "emulsifying surfactant" means an amphiphilic surfactant compound, i.e. one which has two parts of different polarity. Generally, one is lipophilic (soluble or dispersible in an oily phase). The other is hydrophilic (soluble or dispersible in water). The emulsifying surfactants are characterized by the value of their HLB (Hydrophilic Lipophilic Balance), the HLB being the ratio between the hydrophilic part and the lipophilic part in the molecule. The term "HLB" is well known to those skilled in the art and is described, for example, in "The HLB system. A time-saving guide to Emulsifier Selection" (published by ICI Americas Inc., 1984). For the emulsifying surfactants, the HLB generally ranges from 3 to 8 for the preparation of W/O emulsions. The HLB of the surfactant(s) used according to the invention may be determined via the Griffin method or the Davies method.

As examples of W/O emulsifying surfactants, mention may be made of alkyl esters or ethers of sorbitan, of glycerol, of polyol or of sugars; silicone surfactants, such as dimethicone copolyols, such as that having the INCI name

Dimethicone (and) PEG/PPG-18/18 Dimethicone sold under the brand X-22-6711D® by the company Shin-Etsu, the mixture of cyclomethicone and of dimethicone copolyol, sold under the name DC 5225 C® by the company Dow Corning, and alkyldimethicone copolyols such as laurylmethicone copolyol sold under the name Dow Corning 5200 Formulation Aid by the company Dow Corning; cetyl dimethicone copolyol, such as cetyl PEG/PPG-10/1 dimethicone, such as the product sold under the name Abil EM 90® by the company Evonik Goldschmidt, and the mixture of cetyl dimethicone copolyol, of polyglyceryl isostearate (4 mol) and of hexyl laurate, sold under the name Abil WE 09® by the company Goldschmidt. One or more coemulsifiers, which may be chosen advantageously from the group comprising polyol alkyl esters, may also be added thereto.

Mention may also be made of non-silicone emulsifying surfactants, in particular alkyl esters or ethers of sorbitan, of glycerol, of polyol or of sugars.

As polyol alkyl esters, mention may be made especially of polyethylene glycol esters, such as PEG-30 dipolyhydroxystearate, such as the product sold under the name Cithrol DPHS-SO-(MV) by the company Croda.

Examples of glycerol and/or sorbitan esters that may be mentioned include polyglyceryl isostearate (INCI name: Polyglyceryl-4 Isostearate), such as the product sold under the name Isolan GI 34® by the company Evonik Goldschmidt; sorbitan isostearate, such as the product sold under the name Arlacel 987® by the company ICI; sorbitan glyceryl isostearate, such as the product sold under the name Arlacel 986® by the company ICI, the diester of a mixture of isostearic, polyhydroxystearic and sebacic acids with Polyglycerin-4 (INCI name: Polyglyceryl-4 Diisostearate/Polyhydroxystearate/Sebacate), such as the product sold under the name Isolan GPS ® by the company Evonik, and mixtures thereof.

According to a particular form of the invention, the emulsifying surfactant may be chosen from emulsifying silicone elastomers.

The term "silicone elastomer" means a supple, deformable organopolysiloxane that has viscoelastic properties and in particular the consistency of a sponge or a supple sphere. Its modulus of elasticity is such that this material withstands deformation and has limited stretchability and contractability. This material is capable of regaining its original shape after stretching.

The emulsifying silicone elastomer may be chosen from polyoxyalkylenated silicone elastomers and polyglycerolated silicone elastomers, and mixtures thereof.

a) Polyoxyalkylenated silicone elastomers

The polyoxyalkylenated silicone elastomer is a crosslinked organopolysiloxane that may be obtained by a crosslinking addition reaction of diorganopolysiloxane containing at least one hydrogen bonded to silicon and of a polyoxyalkylene containing at least two ethylenically unsaturated groups.

Preferably, the polyoxyalkylenated crosslinked organopolysiloxane is obtained by a crosslinking addition reaction (A1) of diorganopolysiloxane containing at least two hydrogens each bonded to a silicon, and (B1) of polyoxyalkylene containing at least two ethylenically unsaturated groups, in particular in the presence (C1) of a platinum catalyst, as described, for example, in patents US 5 236 986 and US 5 412 004.

In particular, the organopolysiloxane may be obtained by reaction of dimethylvinylsiloxy-terminated polyoxyalkylene (in particular polyoxyethylene and/or polyoxypropylene) and of trimethylsiloxy-terminated methylhydropolysiloxane, in the presence of a platinum catalyst.

The organic groups bonded to the silicon atoms of compound (A1) may be alkyl groups containing from 1 to 18 carbon atoms, such as methyl, ethyl, propyl, butyl, octyl, decyl, dodecyl (or lauryl), myristyl, cetyl or stearyl; substituted alkyl groups such as 2-phenylethyl, 2-phenylpropyl or 3,3,3-trifluoropropyl; aryl groups such as phenyl, tolyl or xylyl; substituted aryl groups such as phenylethyl; and substituted monovalent hydrocarbon-based groups such as an epoxy group, a carboxylate ester group or a mercapto group.

Compound (A1) may thus be chosen from trimethylsiloxy-terminated methylhydropolysiloxanes, trimethylsiloxy-terminated dimethylsiloxane/methylhydrosiloxane copolymers, dimethylsiloxane/methylhydrosiloxane cyclic copolymers, and trimethylsiloxy-terminated dimethylsiloxane/methylhydrosiloxane/laurylmethylsiloxane copolymers.

Compound (C1) is the catalyst for the crosslinking reaction, and is especially chloroplatinic acid, chloroplatinic acid-olefin complexes, chloroplatinic acid-alkenylsiloxane complexes, chloroplatinic acid-diketone complexes, platinum black and platinum on a support.

Advantageously, the polyoxyalkylenated silicone elastomers may be formed from divinyl compounds, in particular polyoxyalkylenes containing at least two vinyl groups, reacting with Si-H bonds of a polysiloxane.

The polyoxyalkylenated silicone elastomer according to the invention is preferably mixed with at least one hydrocarbon-based oil and/or one silicone oil to form a gel. In these gels, the polyoxyalkylenated elastomer may be in the form of non-spherical particles.

Polyoxyalkylenated elastomers are described especially in patents US 5 236 986, US 5 412 004, US 5 837 793 and US 5 811 487.

As polyoxyalkylenated silicone elastomers, use may be made of those having the following INCI names:

Dimethicone /PEG-10/15-Crosspolymer,
PEG-15/Lauryl Dimethicone Crosspolymer,
PEG-10/Lauryl Dimethicone Crosspolymer,
PEG-12 Dimethicone Crosspolymer,
PEG-10 Dimethicone Crosspolymer,
PEG-10 Dimethicone/Vinyl Dimethicone Crosspolymer,
PEG-12 Dimethicone/PPG-20 Crosspolymer, and mixtures thereof.

They are especially sold under the KSG® names by the company Shin-Etsu:

KSG-210® (INCI name: Dimethicone and Dimethicone/PEG-10/15-Crosspolymer;
KSG-310® INCI name: PEG-15/Lauryl Dimethicone Crosspolymer and Mineral oil;
KSG-320® INCI name: PEG-15/Lauryl Dimethicone Crosspolymer and Isododecane;
KSG-330® INCI name: PEG-15/Lauryl Dimethicone Crosspolymer and Triethylhexanoin;
KSG-340® INCI name: Squalane and PEG-15/Lauryl Dimethicone Crosspolymer. They are especially sold by the company Dow Corning under the name Dow Corning 9011 Silicone Elastomer Blend ®; INCI name: Cyclopentasiloxane and PEG-12 Dimethicone Crosspolymer.

Mention may also be made of the product sold under the name Dow Corning EL-7040 Hydro Elastomer Blend ® by the company Dow Corning for the compound which has the INCI name: PEG-12 Dimethicone/PPG-20 Crosspolymer.

b) <u>Polyglycerolated silicone elastomers</u>

The polyglycerolated silicone elastomer is an elastomeric crosslinked organopolysiloxane that may be obtained by a crosslinking addition reaction of diorganopolysiloxane containing at least one hydrogen bonded to silicon and of polyglycerolated compounds containing ethylenically unsaturated groups, in particular in the presence of a platinum catalyst.

Preferably, the elastomeric crosslinked organopolysiloxane is obtained by a crosslinking addition reaction (A) of diorganopolysiloxane containing at least two hydrogens each bonded to a silicon, and (B) of glycerolated compounds containing at least two ethylenically unsaturated groups, in particular in the presence (C) of a platinum catalyst.

In particular, the organopolysiloxane may be obtained by reaction of a dimethylvinylsiloxy-terminated polyglycerolated compound and of trimethylsiloxy-terminated methylhydropolysiloxane, in the presence of a platinum catalyst.

Compound (A) is the base reagent for the formation of organopolysiloxane elastomer, and the crosslinking is performed by addition reaction of compound (A) with compound (B) in the presence of catalyst (C).

Compound (A) is in particular an organopolysiloxane containing at least two hydrogen atoms bonded to different silicon atoms in each molecule.

Compound (A) may have any molecular structure, especially a linear-chain or branched-chain structure or a cyclic structure.

Compound (A) may have a viscosity at 25°C ranging from 1 to 50 000 centistokes, especially so as to be readily miscible with compound (B).

The organic groups bonded to the silicon atoms of compound (A) may be alkyl groups containing from 1 to 18 carbon atoms, such as methyl, ethyl, propyl, butyl, octyl, decyl, dodecyl (or lauryl), myristyl, cetyl or stearyl; substituted alkyl groups such as 2-phenylethyl, 2-phenylpropyl or 3,3,3-trifluoropropyl; aryl groups such as phenyl, tolyl or xylyl; substituted aryl groups such as phenylethyl; and substituted monovalent hydrocarbon-based groups such as an epoxy group, a carboxylate ester group or a mercapto group. Preferably, said organic group is chosen from methyl, phenyl and lauryl groups.

Compound (A) may thus be chosen from trimethylsiloxy-terminated methylhydropolysiloxanes, trimethylsiloxy-terminated dimethylsiloxane/methylhydrosiloxane copolymers, dimethylsiloxane/methylhydrosiloxane cyclic copolymers, and trimethylsiloxy-terminated dimethylsiloxane/methylhydrosiloxane/laurylmethylsiloxane copolymers.

Compound (B) may be a polyglycerolated compound corresponding to formula (B') below:

$$C_mH_{2m-1}\text{ -O-[ Gly ]}_n\text{-}C_mH_{2m-1} \qquad (B')$$

in which m is an integer ranging from 2 to 6, n is an integer ranging from 2 to 200, preferably ranging from 2 to 100, preferably ranging from 2 to 50, preferably n ranging from 2 to 20, preferably ranging from 2 to 10 and preferentially ranging from 2 to 5, and in particular n is equal to 3; Gly denotes:

$-CH_2-CH(OH)-CH_2-O-$

or

$-CH_2-CH(CH_2OH)-O-$

Advantageously, the sum of the number of ethylenic groups per molecule of compound (B) and of the number of hydrogen atoms bonded to silicon atoms per molecule of compound (A) is at least 4.

It is advantageous for compound (A) to be added in an amount such that the molecular ratio between the total amount of hydrogen atoms bonded to silicon atoms in compound (A) and the total amount of all the ethylenically unsaturated groups in compound (B) is within the range from 1/1 to 20/1.

Compound (C) is the catalyst for the crosslinking reaction, and is especially chloroplatinic acid, chloroplatinic acid-olefin complexes, chloroplatinic acid-alkenylsiloxane complexes, chloroplatinic acid-diketone complexes, platinum black and platinum on a support.

Catalyst (C) is preferably added in an amount of from 0.1 to 1000 parts by weight and better still from 1 to 100 parts by weight, as clean platinum metal, per 1000 parts by weight of the total amount of compounds (A) and (B).

The polyglycerolated silicone elastomer according to the invention is generally mixed with at least one hydrocarbon-based oil and/or one silicone oil to form a gel. In these gels, the polyglycerolated elastomer is often in the form of non-spherical particles.

Such elastomers are described especially in patent application WO 2004/024798.

Use may be made, as polyglycerolated silicone elastomers, of the following compounds having the INCI name:

Dimethicone/polyglycerin-3 crosspolymer,
Lauryl dimethicone/polyglycerin-3 crosspolymer,
and mixtures thereof.

They are especially sold by the company Shin-Etsu under the following names:

KSG-710®; INCI name: Dimethicone/polyglycerin-3 crosspolymer and dimethicone; KSG-810®; INCI name: Mineral oil and lauryl dimethicone/polyglycerin-3 crosspolymer;
KSG-820®; INCI name: Isododecane and lauryl dimethicone/polyglycerin-3 crosspolymer;
KSG-830®; INCI name: Triethylhexanoin and lauryl dimethicone/polyglycerin-3 crosspolymer;
KSG-840®; INCI name: Squalane and lauryl dimethicone/polyglycerin-3 crosspolymer.

According to a particular form of the invention, the following will be chosen as emulsifiers:

- PEG-30 dipolyhydroxystearate;
- and Polyglyceryl-4 diisostearate/polyhydroxystearate/sebacate;
- and mixtures thereof.

**ADDITIVES**

The compositions according to the invention may in addition comprise additives commonly used in care and/or makeup products, such as

- active agents such as vitamins, for example vitamins A, E, C, $B_3$; baicalin or plant extracts containing same, in particular of root of Chinese skullcap and in particular *Scutellaria baicalensis* (INCI name: *Scutellaria baicalensis* root extract); moisturizers;
- sunscreens other than the β,β-diphenylacrylate and salicylate organic UV-screening agents as described previously;
- fillers;
- additional dyestuffs;
- and mixtures thereof.

It is a matter of routine operation for those skilled in the art to adjust the nature and the amount of the additives present in the compositions in accordance with the invention such that the desired cosmetic properties thereof are not thereby affected.

**a) Additional UV-screening agents**

The compositions according to the invention may also contain one or more additional UV-screening agents chosen from organic UV-screening agents and/or mineral screening agents.

i) Organic UV-screening agents

The organic UV-screening agents are especially chosen from cinnamic compounds; dibenzoylmethane compounds; anthranilate compounds; benzylidenecamphor compounds; benzophenone compounds; triazine compounds; benzotriazole compounds, especially the silicone benzotriazoles described in patent EP0392883 and the methylenebis(hydroxyphenyl benzotriazole) compounds as described in applications US 5 237 071, US 5 166 355, GB2303549, DE 197 26 184 and EP893119; benzalmalonate compounds, especially those mentioned in patent US 5 624 663; benzimidazole derivatives; imidazoline compounds; the bis-benzoazolyl compounds as described in patents EP669323 and US 2 463 264; the benzoxazole compounds as described in patent applications EP0832642, EP1027883, EP1300137 and DE10162844; screening polymers and screening silicones such as those described in particular in application WO-93/04665; the merocyanine compounds as described in US 4 195 999, patent application WO2004/006878, patent applications WO2008/090066, WO2011113718, WO2009027258, WO2013010590, WO2013011094, WO2013011480 and documents IP COM JOURNAL N°000179675D published on 23 February 2009, IP COM JOURNAL N°000182396D published on 29 April 2009, IP COM JOURNAL N° 000189542D published on 12 November 2009, and IP COM Journal N°IPCOM000011179D published on 04/03/2004, and mixtures thereof.

As examples of organic photoprotective agents, mention may be made of those denoted hereinbelow under their INCI name:

Dibenzoylmethane compounds

Butyl methoxydibenzoylmethane sold especially under the trade name Parsol 1789® by DSM Nutritional Products, Inc.;

Cinnamic compounds:

Ethylhexyl methoxycinnamate, sold especially under the trade name Parsol MCX® by DSM Nutritional Products,

Isopropyl methoxycinnamate,
Isoamyl p-methoxycinnamate sold under the trade name Neo Heliopan E 1000® by Symrise,

Benzophenone compounds:

Benzophenone-1 sold under the trade name Uvinul 400® by BASF,
Benzophenone-2, sold under the trade name Uvinul D 50® by BASF,
Benzophenone-3 or Oxybenzone, sold under the trade name Uvinul M 40® by BASF,
Benzophenone-4, sold under the trade name Uvinul MS 40® by BASF,
Benzophenone-5,
Benzophenone-6 sold under the trade name Helisorb 11® by Norquay,
Benzophenone-8, sold under the trade name Spectra-Sorb UV-24® by American Cyanamid,
Benzophenone-9, sold under the trade name Uvinul DS 49® by BASF, Benzophenone-12,
n-Hexyl 2-(4-diethylamino-2-hydroxybenzoyl)benzoate, sold under the trade name Uvinul A Plus® or, as a mixture with octyl methoxycinnamate, under the trade name Uvinul A Plus B® by the company BASF,
1,1'-(1,4-Piperazinediyl)bis[1-[2-[4-(diethylamino)-2-hydroxybenzoyl]phenyl]methanone] (CAS 919803-06-8), as described in patent application WO 2007/071 584; this compound advantageously being used in micronized form (mean size of 0.02 to 2 μm), which may be obtained, for example, according to the micronization process described in patent applications GB-A-2 303 549 and EP-A-893 119, and in particular in the form of an aqueous dispersion.

Benzylidenecamphor compounds:

3-Benzylidenecamphor, manufactured under the name Mexoryl SD® by Chimex, 4-Methylbenzylidenecamphor, sold under the name Eusolex 6300® by Merck,
Benzylidenecamphorsulfonic acid, manufactured under the name Mexoryl SL® by Chimex,
Camphor benzalkonium methosulfate, manufactured under the name Mexoryl SO® by Chimex,
Terephthalylidenedicamphorsulfonic acid, manufactured under the name Mexoryl SX® by Chimex,
Polyacrylamidomethylbenzylidenecamphor, manufactured under the name Mexoryl SW® by Chimex.

Phenylbenzimidazole compounds:

Phenylbenzimidazolesulfonic acid, sold in particular under the trade name Eusolex 232® by Merck.

Bis-benzazolyl compounds:

Disodium phenyl dibenzimidazole tetrasulfonate, sold under the trade name Neo Heliopan AP® by Symrise.

Benzotriazole compounds

Drometrizole trisiloxane, manufactured under the name Mexoryl SX® by Chimex; Methylene bis-benzotriazolyl tetramethylbutylphenol in particular in solid form, such as the product sold under the trade name Mixxim BB/100® by Fairmount Chemical or in the form of an aqueous dispersion of micronized particles having a mean particle size which ranges from 0.01 to 5 μm and more preferentially from 0.01 to 2 μm and more particularly from 0.020 to 2 μm, with at least one alkylpolyglycoside surfactant of structure:
$CnH_{2n+1}\ O(C_6H_{10}O_5)_xH$ in which n is an integer from 8 to 16 and x is the average degree of polymerization of the $(C_6H_{10}O_5)$ unit and ranges from 1.4 to 1.6, as described in patent GB-A-2 303 549, in particular sold under the trade name Tinosorb M® by the company BASF or in the form of an aqueous dispersion of micronized particles having a mean particle size which ranges from 0.02 to 2 μm and more preferentially from 0.01 to 1.5 μm and more particularly from 0.02 to 1 μm in the presence of at least one mono-$(C_8\text{-}C_{20})$alkyl ester of polyglycerol having a degree of glycerol polymerization of at least 5, such as the aqueous dispersions described in application WO 2009/063392.

Triazine compounds:

- Bis-Ethylhexyloxyphenol methoxyphenyl triazine, sold under the trade name Tinosorb S® by BASF,
- Ethylhexyl triazone, sold in particular under the trade name Uvinul T150® by BASF,
- Diethylhexyl butamido triazone, sold under the trade name Uvasorb HEB® by Sigma 3V,
- symmetrical triazine screening agents substituted with naphthalenyl groups or polyphenyl groups described in patent US 6 225 467, patent application WO 2004/085 412 (see compounds 6 and 9) or the document "Symmetrical Triazine Derivatives", IP.COM IPCOM000031257 Journal, INC, West Henrietta, NY, US (20 September 2004), especially 2,4,6-tris(diphenyl)triazine and 2,4,6-tris(terphenyl)triazine, which is also mentioned in patent applications WO 06/035 000, WO 06/034 982, WO 06/034 991, WO 06/035 007, WO 2006/034 992 and WO 2006/034 985, these compounds advantageously being used in micronized form (mean particle size of 0.02 to 3 μm), which may be obtained, for example, according to the micronization process described in patent applications GB-A-2 303 549 and EP-A-893 119, and especially in aqueous dispersion;
- silicone triazines substituted with two aminobenzoate groups, as described in patent EP 0 841 341, in particular 2,4-bis(n-butyl 4’-aminobenzalmalonate)-6-[(3-{1,3,3,3-tetramethyl-1-[(trimethylsilyl)oxy]disiloxanyl}propyl)amino]-s-triazine.

Anthranilic compounds:

Menthyl anthranilate, sold under the trade name Neo Heliopan MA® by Symrise.

Imidazoline compounds:

Ethylhexyl dimethoxybenzylidene dioxoimidazoline propionate,

Benzalmalonate compounds:

Polyorganosiloxane containing benzalmalonate functional groups, for instance Polysilicone-15, sold under the trade name Parsol SLX® by DSM Nutritional Products, Inc.

Benzoxazole compounds:

2,4-bis[5-(1,1-dimethylpropyl)benzoxazol-2-yl(4-phenyl)imino]-6-(2-ethylhexyl)imino-1,3,5-triazine, sold under the name Uvasorb K2A® by Sigma 3V.

ii) Mineral UV-screening agents

The mineral UV-screening agents used in accordance with the present invention are metal oxide pigments. More preferentially, the mineral UV-screening agents of the invention are metal oxide particles with a mean elementary particle size of less than or equal to 0.5 $\mu$m, more preferentially between 0.005 and 0.5 $\mu$m, even more preferentially between 0.01 and 0.2 $\mu$m, better still between 0.01 and 0.1 $\mu$m and more particularly between 0.015 and 0.05 $\mu$m.

They may be chosen in particular from titanium oxide, zinc oxide, iron oxide, zirconium oxide and cerium oxide, or mixtures thereof.

Such coated or uncoated metal oxide pigments are described in particular in patent application EP-A-0 518 773. Commercial pigments that may be mentioned include the products sold by the companies Sachtleben Pigments, Tayca, Merck and Degussa.

The metal oxide pigments may be coated or uncoated.

The coated pigments are pigments that have undergone one or more surface treatments of chemical, electronic, mechanochemical and/or mechanical nature with compounds such as amino acids, beeswax, fatty acids, fatty alcohols, anionic surfactants, lecithins, sodium, potassium, zinc, iron or aluminium salts of fatty acids, metal alkoxides (of titanium or aluminium), polyethylene, silicones, proteins (collagen, elastin), alkanolamines, silicon oxides, metal oxides or sodium hexametaphosphate.

The coated pigments are more particularly titanium oxides that have been coated:

- with silica, such as the product Sunveil® from the company Ikeda,
- with silica and iron oxide, such as the product Sunveil F® from the company Ikeda,
- with silica and alumina, such as the products Microtitanium Dioxide MT 500 SA® and Microtitanium Dioxide MT 100 SA from the company Tayca and Tioveil from the company Tioxide,
- with alumina, such as the products Tipaque TTO-55 (B)® and Tipaque TTO-55 (A)® from the company Ishihara and UVT 14/4 from the company Sachtleben Pigments,
- with alumina and aluminium stearate, such as the products Microtitanium Dioxide MT 100 T®, MT 100 TX®, MT 100 Z® and MT-01® from the company Tayca, the products Solaveil CT-10 W® and Solaveil CT 100® from the company Uniqema and the product Eusolex T-AVO® from the company Merck,
- with silica, alumina and alginic acid, such as the product MT-100 AQ® from the company Tayca,
- with alumina and aluminium laurate, such as the product Microtitanium Dioxide MT 100 S® from the company Tayca,
- with iron oxide and iron stearate, such as the product Microtitanium Dioxide MT 100 F® from the company Tayca,
- with zinc oxide and zinc stearate, such as the product BR 351® from the company Tayca,
- with silica and alumina and treated with a silicone, such as the products Microtitanium Dioxide MT 600 SAS®, Microtitanium Dioxide MT 500 SAS® or Microtitanium Dioxide MT 100 SAS® from the company Tayca,
- with silica, alumina and aluminium stearate and treated with a silicone, such as the product STT-30-DS® from the company Titan Kogyo,
- with silica and treated with a silicone, such as the product UV-Titan X 1950 from the company Sachtleben Pigments,
- with alumina and treated with a silicone, such as the products Tipaque TTO-55 (S)® from the company Ishihara or UV Titan M 262® from the company Sachtleben Pigments,
- with triethanolamine, such as the product STT-65-S from the company Titan Kogyo,
- with stearic acid, such as the product Tipaque TTO-55 (C)® from the company Ishihara,
- with sodium hexametaphosphate, such as the product Microtitanium Dioxide MT 150 W® from the company Tayca,
- $TiO_2$ treated with octyltrimethylsilane, sold under the trade name T 805® by the company Degussa Silices,
- $TiO_2$ treated with a polydimethylsiloxane, sold under the trade name 70250 Cardre UF TiO2SI3® by the company Cardre,
- anatase/rutile $TiO_2$ treated with a polydimethylhydrogenosiloxane, sold under the trade name Microtitanium Dioxide USP Grade Hydrophobic® by the company Color Techniques.

Mention may also be made of $TiO_2$ pigments doped with at least one transition metal such as iron, zinc or manganese and more particularly manganese. Preferably, said doped pigments are in the form of an oily dispersion. The oil present in the oily dispersion is preferably chosen from triglycerides including those of capric/caprylic acids. The oily dispersion of titanium oxide particles may also comprise one or more dispersants, for instance a sorbitan ester, for instance sorbitan isostearate, or a polyoxyalkylenated fatty acid ester of glycerol, for instance TRI-PPG-3 myristyl ether citrate and polyglyceryl-3 polyricinoleate. Preferably, the oily dispersion of titanium oxide particles comprises at least one dispersant chosen from polyoxyalkylenated fatty acid esters of glycerol. Mention may be made more particularly of the oily dispersion of $TiO_2$ particles doped with manganese in capric/caprylic acid triglyceride in the presence of TRI-PPG-3 myristyl ether citrate and polyglyceryl-3 polyricinoleate and sorbitan isostearate having the INCI name: titanium dioxide (and) TRI-PPG-3 myristyl ether citrate (and) polyglyceryl-3 ricinoleate (and) sorbitan isostearate, for instance the product sold under the trade name Optisol TD50® by the company Croda.

The uncoated titanium oxide pigments are sold, for example, by the company Tayca under the trade names Microtitanium Dioxide MT 500 B or Microtitanium Dioxide MT 600 B®, by the company Degussa under the name P 25, by the company Wackherr under the name Transparent titanium oxide PW®, by the company Miyoshi Kasei under the name UFTR®, by the company Tomen under the name ITS® and by the company Tioxide under the name Tioveil AQ.

The uncoated zinc oxide pigments are, for example:

- those sold under the name Z-Cote by Sunsmart;
- those sold under the name Nanox® by the company Elementis;
- those sold under the name Nanogard WCD 2025® by the company Nanophase Technologies.

The coated zinc oxide pigments are, for example:

- those sold under the name Zinc Oxide CS-5® by the company Toshibi (ZnO coated with polymethylhydrogenosiloxane);
- those sold under the name Nanogard Zinc Oxide FN® by Nanophase Technologies (as a 40% dispersion in Finsolv TN®, C12-C15 alkyl benzoate);
- those sold under the name Daitopersion Zn-30® and Daitopersion Zn-50® by Daito (dispersions in cyclopolymethylsiloxane/oxyethylenated polydimethylsiloxane, containing 30% or 50% of zinc oxides coated with silica and polymethylhydrogenosiloxane);
- those sold under the name NFD Ultrafine ZnO® by the company Daikin (ZnO coated with perfluoroalkyl phosphate and copolymer based on perfluoroalkylethyl as a dispersion in cyclopentasiloxane);
- those sold under the name SPD-Z1® by the company Shin-Etsu (ZnO coated with silicone-grafted acrylic polymer, dispersed in cyclodimethylsiloxane);
- those sold under the name Escalol Z100® by the company ISP (alumina-treated ZnO dispersed in the ethylhexyl methoxycinnamate/PVP-hexadecene copolymer/methicone mixture);
- those sold under the name Fuji ZnO-SMS-10® by the company Fuji Pigment (ZnO coated with silica and polymethylsilsesquioxane);
- those sold under the name Nanox Gel TN® by the company Elementis (ZnO dispersed at a concentration of 55% in $C_{12}$-$C_{15}$ alkyl benzoate with hydroxystearic acid polycondensate).

The uncoated cerium oxide pigments may be, for example, those sold under the name Colloidal Cerium Oxide® by the company Rhône-Poulenc.

The uncoated iron oxide pigments are sold, for example, by the company Arnaud under the names Nanogard WCD 2002® (FE 45B®), Nanogard Iron FE 45 BL AQ, Nanogard FE 45R AQ® and Nanogard WCD 2006® (FE 45R®) or by the company Mitsubishi under the name TY-220®.

The coated iron oxide pigments are sold, for example, by the company Arnaud under the names Nanogard WCD 2008 (FE 45B FN)®, Nanogard WCD 2009® (FE 45B 556®), Nanogard FE 45 BL 345® and Nanogard FE 45 BL® or by the company BASF under the name Transparent Iron Oxide®.

Mention may also be made of mixtures of metal oxides, especially of titanium dioxide and of cerium dioxide, including the equal-weight mixture of titanium dioxide and cerium dioxide coated with silica, sold by the company Ikeda under the name Sunveil A®, and also the mixture of titanium dioxide and zinc dioxide coated with alumina, silica and silicone, such as the product M 2610 sold by the company Sachtleben Pigments, or coated with alumina, silica and glycerol, such as the product M 2110 sold by the company Sachtleben Pigments.

According to the invention, the coated or uncoated mineral screening agents based on titanium oxide are particularly preferred.

b) Fillers

The compositions in accordance with the invention may also comprise at least one filler, of organic or mineral nature, which can especially give them complementary properties of mattness, coverage, persistence and/or improved stability.

The term "filler" should be understood as meaning colourless or white solid particles of any form, which are in an insoluble form dispersed in the medium of the composition. These particles, of mineral or organic nature, give body or rigidity to the composition and/or softness and uniformity to the makeup.

The fillers used in the compositions according to the invention may be of lamellar, globular, spherical or fibrous form or of any other form intermediate between these defined forms.

The fillers according to the invention may or may not be surface-coated, and in particular they may be surface-treated with silicones, amino acids, fluorinated derivatives or any other substance which promotes the dispersion and the compatibility of the filler in the composition.

Examples of mineral fillers that may be mentioned include talc, mica, silica, hollow silica microspheres, kaolin, calcium carbonate, magnesium carbonate, hydroxyapatite, boron nitride, glass or ceramic microcapsules, or composites of silica and of titanium dioxide, for instance the TSG series sold by Nippon Sheet Glass.

Examples of organic fillers that may be mentioned include polyamide powders (Nylon® Orgasol from Atochem), polyethylene powders, polymethyl methacrylate powders, polytetrafluoroethylene (Teflon) powders, acrylic acid copolymer powders (Polytrap from the company Dow Corning), lauroyl lysine, hollow polymer microspheres such as those of polyvinylidene chloride/acrylonitrile, for instance Expancel (Nobel Industrie), hexamethylene diisocyanate/trimethylol hexyllactone copolymer powder (Plastic Powder from Toshiki), silicone resin microbeads (for example Tospearl from Toshiba), synthetic or natural micronized waxes, metal soaps derived from organic carboxylic acids containing from 8 to 22 carbon atoms and preferably from 12 to 18 carbon atoms, for example zinc stearate, magnesium stearate, lithium stearate, zinc laurate or magnesium myristate, Polypore® L 200 (Chemdal Corporation), powders of crosslinked elastomeric organopolysiloxane coated with silicone resin, especially with silsesquioxane resin, as described, for example, in patent US 5 538 793, polyurethane powders, in particular powders of crosslinked polyurethane comprising a copolymer, said copolymer comprising trimethylol hexyllactone. It may in particular be a hexamethylene diisocyanate/trimethylol hexyllactone polymer. Such particles are especially commercially available, for example, under the name Plastic Powder D-400® or Plastic Powder D-800® from the company Toshiki, and mixtures thereof. It may also be cellulose powder, such as the product sold by Daito in the Cellulobeads range.

According to a particular embodiment of the invention, the composition comprises at least one crosslinked elastomeric organopolysiloxane powder coated with silicone resin.

The term "organopolysiloxane elastomer" or "silicone elastomer" means a supple, crosslinked, deformable organopolysiloxane having viscoelastic properties and especially with the consistency of a sponge or a supple sphere. Its modulus of elasticity is such that this material withstands deformation and has limited stretchability and contractability. This material is capable of regaining its original shape after stretching.

Such elastomer powders are sold under the names KSP-100®, KSP-101®, KSP-1020, KSP-103®, KSP-104® and KSP-105® by the company Shin-Etsu, and have the INCI name: vinyl dimethicone/methicone silsesquioxane crosspolymer.

Mention may also be made of:

- crosslinked elastomeric organopolysiloxane powders coated with silicone resin, such as powders of hybrid silicone functionalized with fluoroalkyl groups, in particular sold under the name KSP-200® by the company Shin-Etsu and having the INCI name: Trifluoropropyl Dimethicone/Vinyl Trifluoropropyldimethicone/Silsesquioxane Crosspolymer; or
- powders of hybrid silicones functionalized with phenyl groups, especially sold under the name KSP-300® by the company Shin-Etsu and having the INCI name: Diphenyl Dimethicone/Vinyl Diphenyl Dimethicone/Silsesquioxane Crosspolymer.

According to an advantageous embodiment, the compositions according to the invention comprise an organopolysiloxane elastomer powder coated with silsesquioxane resin, having the INCI name Vinyl Dimethicone/Methicone Silsesquioxane Crosspolymer, such as those sold under the names KSP-100®.

The organopolysiloxane elastomer powder(s) coated with a silicone resin may be present in a content ranging from 0.5% to 12% by weight, advantageously from 1% to 8% by weight, relative to the total weight of said composition.

c) Additional dyestuffs

A composition according to the invention may also comprise at least one additional dyestuff, preferably in a

proportion of at least 0.01% by weight relative to the total weight of the composition.

For obvious reasons, this amount is liable to vary significantly with regard to the intensity of the desired colour effect and of the colour intensity afforded by the dyestuffs under consideration, and its adjustment clearly falls within the competence of a person skilled in the art.

The additional dyestuffs that are suitable for use in the invention may be water-soluble, but may also be liposoluble.

For the purposes of the invention, the term "*water-soluble dyestuff*" means any natural or synthetic, generally organic compound, which is soluble in an aqueous phase or water-miscible solvents and which is capable of imparting colour.

As water-soluble dyes that are suitable for use in the invention, mention may be made especially of synthetic or natural water-soluble dyes, for instance FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1, betanine (beetroot), carmine, copper chlorophylline, methylene blue, anthocyanins (enocianin, black carrot, hibiscus and elder), caramel and riboflavin.

The water-soluble dyes are, for example, beetroot juice and caramel.

For the purposes of the invention, the term "*liposoluble dyestuff*" means any natural or synthetic, generally organic compound, which is soluble in an oily phase or in solvents that are miscible with a fatty substance, and which is capable of imparting colour.

As liposoluble dyes that are suitable for use in the invention, mention may be made in particular of synthetic or natural liposoluble dyes, for instance DC Red 17, DC Red 21, DC Red 27, DC Green 6, DC Yellow 11, DC Violet 2, DC Orange 5, Sudan red, carotenes (β-carotene, lycopene), xanthophylls (capsanthin, capsorubin, lutein), palm oil, Sudan brown, quinoline yellow, annatto and curcumin.

## COSMETIC COMPOSITIONS

The present invention also relates to a cosmetic composition comprising, in a physiologically acceptable medium, a composition as defined above.

The term "physiologically acceptable medium" is intended to denote a medium that is particularly suitable for the application of a composition according to the invention to the skin.

The physiologically acceptable medium is generally adapted to the nature of the support onto which the composition has to be applied, and also to the appearance under which the composition has to be packaged.

## APPLICATIONS

According to one embodiment, a composition of the invention may advantageously be in the form of a composition for caring for the skin and/or keratin fibres, the body or the face, in particular the face.

According to another embodiment, a composition of the invention may advantageously be in the form of a composition for making up keratin materials, in particular the skin of the body or of the face, in particular of the face.

Thus, according to a sub-mode of this embodiment, a composition of the invention may advantageously be in the form of a makeup base composition.

A composition of the invention may advantageously be in the form of a foundation.

According to another sub-mode of this embodiment, a composition of the invention may advantageously be in the form of a composition for making up the skin and especially the face. It may thus be an eyeshadow or a face powder.

Such compositions are especially prepared according to the general knowledge of a person skilled in the art.

Throughout the description, including the claims, the term "*comprising a*" should be understood as being synonymous with "*comprising at least one*", unless otherwise specified.

The terms "*between... and...*" and "*ranging from... to...*" should be understood as being inclusive of the limits, unless otherwise specified.

The invention is illustrated in greater detail by the examples and figures presented below. Unless otherwise indicated, the amounts shown are expressed as weight percentages.

## EXAMPLE: Foundation in the form of a water/oil emulsion

| Phase | Ingredients | EX. 1 (invention) |
|---|---|---|
| A1 | Polyglyceryl-4 diisostearate/polyhydroxystearate/ sebacate (Isolan GPS® from Evonik) | 0.75 |
| | PEG-30 dipolyhydroxystearate (Cithrol DPHS-SO-(MV) from the company CRODA) | 1.00 |
| A2 | Homosalate | 10 |
| | Ethylhexyl salicylate | 5 |
| | Octocrylene | 7 |
| A3 | Dodecamethylpentasiloxane PDMS 2CST (Silicone Fluid 2CS® from Dow Corning) | 16.00 |
| A4 | Disteardimonium hectorite (Bentone V38® from Elementis) | 0.75 |
| A5 | Vinyl dimethicone/methicone silsesquioxane crosspolymer (KSP 100® from Shin-Etsu) | 2.00 |
| A6 | Vitamin E tocopherol | 1.00 |
| B | Propanediol | 5 |
| | *Scutellaria baicalensis* root extract (Baicalin 95 MM® from MMP) | 0.2 |
| | Magnesium sulfate | 0.7000 |
| | Water | qs 100 |
| | Phenoxyethanol | 0.50 |
| | Niacinamide | 2 |
| | Caffeine | 1 |
| C | Alcohol | 5 |
| D | Yellow iron oxide coated with aluminium stearoyl glutamate<br>Red iron oxide coated with aluminium stearoyl glutamate<br>Black iron oxide coated with aluminium stearoyl glutamate<br>Titanium dioxide coated with aluminium stearoyl glutamate | 14 |

**Preparation protocol**

Preparation of the aqueous phase B:

In a glass beaker: the niacinamide was dissolved in water with stirring using a magnetic bar, the caffeine was then added to obtain a transparent solution, the baicalin was then added to obtain a clear yellow transparent solution with a pH of 4.7, and the $MgSO_4$ salt, propanediol and phenoxyethanol were then added to obtain a clear yellow transparent solution with a pH of 4.75.

Preparation of the oily phase A:

In a capsule, a part of the silicone (twice the amount of clay) was taken to wet the clay with a flexible spatula,

- on a hot plate, in the main beaker, the ethylhexyl salicylate was heated with the PEG-30 dipolyhydroxystearate and the Isolan GPS at about 55°C, until the PEG-30 dipolyhydroxystearate had melted, and the mixture was then cooled by adding the other two sunscreens (the temperature drops to about 30°C) and the rest of the silicone was added.

Emulsification:

The aqueous phase was added little by little, with stirring using a Moritz blender, to the oily phase to obtain a concentrated white base, the stirring speed was increased and the mixture was left stirring for 10 minutes until a fluid mixture was obtained. The mixture of modified clay and volatile silicone oil was added to the white base and the resulting mixture was stirred in a turbine very vigorously to activate the clay, and was left stirring for 15 minutes until the mixture had thickened. The KSP 100 was added, with stirring using a Moritz blender, and the mixture was left for 10 minutes until it had thickened. The vitamin E and then the pigments were added until there was good development of the colour. The formula was then debubbled.

Protocol for evaluating *in vitro* the SPF screening efficiency

The sun protection factor was determined according to the *in vitro* method described by B.L. Diffey in J. Soc. Cosmet. Chem. 40, 127-133, (1989). The measurements were taken using a UV-2000 spectrophotometer from the company Labsphere. The composition was applied to a rough plate of PMMA, in the form of a uniform and even deposit in a proportion of 1 mg/$cm^2$.

The *in vitro* sun protection factor SPF is 78.91 ± 19.85.

The composition obtained offers good photoprotection (SPF > 30), is stable at room temperature (20-25°C) and has a uniform appearance (good miscibility of the octocrylene in the non-cyclic volatile silicone oil). When applied to the skin, good glidance and good sensory properties such as absence of a greasy effect, no film sensation on the skin and a lightness effect are observed.

**Claims**

**1.** Composition, especially comprising a physiologically acceptable medium, especially for coating keratin materials, more particularly for making up and/or caring for keratin materials such as the skin, containing at least:

a) an oily continuous phase, and
b) at least one mixture of liquid organic UV-screening agents comprising:

(i) at least one UV-screening agent of the β,β-diphenylacrylate type, and
(ii) at least one UV-screening agent of the salicylate type; and

c) at least one non-cyclic volatile silicone oil; and
d) at least one pigment; the said composition being :

i) an anhydrous composition comprising a single oily phase, or
ii) a water-in-oil emulsion.

**2.** Composition according to Claim 1, in which the concentration of said mixture of liquid organic UV-screening agents is at least 15% by weight, and more preferentially at a concentration of at least 20% by weight, relative to the total weight of the composition.

**3.** Composition according to Claim 1 or 2, in which the concentration of said mixture of liquid organic UV-screening agents ranges from 15% to 40% by weight, and more preferentially ranging from 20% to 30% by weight, relative to the total weight of the composition.

**4.** Composition according to any one of the preceding claims, in which the UV-screening agent of the β,β-diphenylacrylate type is the compound 2-ethylhexyl 2-cyano-3,3-diphenylacrylate, or Octocrylene.

**5.** Composition according to any one of the preceding claims, in which the UV-screening agent of the salicylate type is chosen from:

- Homosalate,
- Ethylhexyl salicylate,
- and mixtures thereof.

**6.** Composition according to any one of the preceding claims, in which the total concentration of oily phase of the composition of the invention preferably ranges from 30% to 95% by weight and more particularly ranging from 40% to 70% by weight relative to the total weight of the composition.

**7.** Composition according to any one of the preceding claims, in which the non-cyclic volatile silicone oils are chosen from:

- the non-cyclic linear silicones of formula (II):

$$R_3SiO\text{-}(R_2SiO)_n\text{-}SiR_3 \qquad (II)$$

in which R, which may be identical or different, denotes:

- a saturated or unsaturated hydrocarbon-based radical, containing from 1 to 10 carbon atoms and preferably from 1 to 6 carbon atoms, optionally substituted with one or more fluorine atoms or with one or more hydroxyl groups, or
- a hydroxyl group,

one of the radicals R possibly being a phenyl group,
n is an integer ranging from 0 to 8, preferably ranging from 2 to 6 and better still ranging from 3 to 5, the silicone compound of formula (II) containing not more than 15 carbon atoms,

- the branched silicones of formula (III) or (IV) below:

$$R_3SiO\text{-}[(R_3SiO)RSiO]\text{-}(R_2SiO)x\text{-}SiR_3 \qquad (III)$$

$$[R_3SiO]_4Si \qquad (IV)$$

in which R, which may be identical or different, denotes:

- a saturated or unsaturated hydrocarbon-based radical, containing from 1 to 10 carbon atoms, optionally substituted with one or more fluorine atoms or with one or more hydroxyl groups, or
- a hydroxyl group,

one of the radicals R possibly being a phenyl group,
x is an integer ranging from 0 to 8,
the silicone compound of formula (III) or (IV) containing not more than 15 carbon atoms.

**8.** Composition according to any one of the preceding claims, in which the non-cyclic volatile silicone oils have a viscosity at 25°C ranging from 0.5 to 8 centistokes (from 0.5 to 8 $mm^2/s$).

**9.** Composition according to any one of the preceding claims, in which the non-cyclic volatile silicone oils are linear, and more particularly chosen from octamethyltrisiloxane, decamethyltetrasiloxane and dodecamethylpentasiloxane, and mixtures thereof, and more particularly dodecamethylpentasiloxane.

**10.** Composition according to any one of the preceding claims, in which the non-cyclic volatile silicone oil/mixture of β,β-diphenylacrylate/salicylate screening agents weight ratio is greater than or equal to 0.5, more preferentially greater than or equal to 0.7, more particularly from 0.5 to 3 and better still from 0.7 to 1.5.

**11.** Composition according to any one of the preceding claims, comprising at least 5% by weight of pigments, more preferentially from 5% to 25% by weight of pigments, in particular from 10% to 30% by weight and preferably from 10% to 20% by weight of pigments, relative to the total weight of said composition.

**12.** Composition according to any one of the preceding claims, comprising at least one pigment coated with at least one lipophilic or hydrophobic compound, in particular hydrophobic coated pigments of iron oxide and/or of titanium dioxide.

**13.** Composition according to any one of Claims 1 to 12, in the form of an anhydrous composition.

**14.** Composition according to any one of Claims 1 to 12, in the form of a water-in-oil emulsion.

**15.** Composition according to any one of the preceding claims, which is in the form of a foundation.

## Patentansprüche

1. Zusammensetzung, insbesondere umfassend ein physiologisch unbedenkliches Medium, insbesondere zum Beschichten von Keratinmaterialien, spezieller zum Schminken und/oder Pflegen von Keratinmaterialien wie der Haut, mindestens enthaltend:

    a) eine ölige kontinuierliche Phase und
    b) mindestens eine Mischung von flüssigen organischen UV-Filtersubstanzen, umfassend:

        (i) mindestens eine UV-Filtersubstanz vom β,β-Diphenylacrylat-Typ und
        (ii) mindestens eine UV-Filtersubstanz vom Salicylat-Typ; und

    c) mindestens ein acyclisches flüchtiges Silikonöl und
    d) mindestens ein Pigment; wobei es sich bei der Zusammensetzung um

        i) eine wasserfreie Zusammensetzung, die eine einzige ölige Phase umfasst, oder
        ii) eine Wasser-in-Öl-Emulsion

    handelt.

2. Zusammensetzung nach Anspruch 1, wobei die Konzentration der Mischung von flüssigen organischen UV-Filtersubstanzen mindestens 15 Gew.-% und weiter bevorzugt mindestens 20 Gew.-%, bezogen auf das Gesamtgewicht der Zusammensetzung, beträgt.

3. Zusammensetzung nach Anspruch 1 oder 2, wobei die Konzentration der Mischung von flüssigen organischen UV-Filtersubstanzen im Bereich von 15 bis 40 Gew.-% und weiter bevorzugt im Bereich von 20 bis 30 Gew.-%, bezogen auf das Gesamtgewicht der Zusammensetzung, liegt.

4. Zusammensetzung nach einem der vorhergehenden Ansprüche, wobei es sich bei der UV-Filtersubstanz vom β,β-Diphenylacrylat-Typ um die Verbindung 2-Ethylhexyl-2-cyano-3,3-diphenylacrylat oder Octocrylene handelt.

5. Zusammensetzung nach einem der vorhergehenden Ansprüche, wobei die UV-Filtersubstanz vom Salicylat-Typ aus

    - Homosalat
    - Ethylhexylsalicylat
    - und Mischungen davon

    ausgewählt ist.

6. Zusammensetzung nach einem der vorhergehenden Ansprüche, wobei die Gesamtkonzentration der öligen Phase der erfindungsgemäßen Zusammensetzung vorzugsweise im Bereich von 30 bis 95 Gew.-% und spezieller im Bereich von 40 bis 70 Gew.-%, bezogen auf das Gesamtgewicht der Zusammensetzung, liegt.

7. Zusammensetzung nach einem der vorhergehenden Ansprüche, wobei die acyclischen flüchtigen Silikonöle aus

    - den acyclischen linearen Silikonen der Formel (II) :

    $$R_3SiO-(R_2SiO)_n-SiR_3 \qquad (II)$$

    wobei R, das gleich oder verschieden sein kann,
    - einen gesättigten oder ungesättigten Rest auf Kohlenwasserstoffbasis mit 1 bis 10 Kohlenstoffatomen und vorzugsweise 1 bis 6 Kohlenstoffatomen, der gegebenenfalls durch ein oder mehrere Fluoratome oder durch ein oder mehrere Hydroxylgruppen substituiert ist, oder
    - eine Hydroxylgruppe

        bedeutet, wobei einer der Reste R gegebenenfalls für eine Phenylgruppe steht,
        n eine ganze Zahl im Bereich von 0 bis 8, vorzugsweise im Bereich von 2 bis 6 und noch besser im Bereich von 3 bis 5, ist, wobei die Silikonverbindung der Formel (II) nicht mehr als 15 Kohlenstoffatomen enthält,

- den verzweigten Silikonen der nachstehenden Formel (III) oder (IV):

$R_3SiO-[(R_3SiO)RSiO]-(R_2SiO)_x-SiR_3$ (III)

$[R_3SiO]_4Si$ (IV)

wobei R, das gleich oder verschieden sein kann,
- einen gesättigten oder ungesättigten Rest auf Kohlenwasserstoffbasis mit 1 bis 10 Kohlenstoffatomen, der gegebenenfalls durch ein oder mehrere Fluoratome oder durch ein oder mehrere Hydroxylgruppen substituiert ist, oder
- eine Hydroxylgruppe

bedeutet, wobei einer der Reste R gegebenenfalls für eine Phenylgruppe steht,
x eine ganze Zahl im Bereich von 0 bis 8 ist, wobei die Silikonverbindung der Formel (III) oder (IV) nicht mehr als 15 Kohlenstoffatome enthält, ausgewählt sind.

**8.** Zusammensetzung nach einem der vorhergehenden Ansprüche, wobei die acyclischen flüchtigen Silikonöle eine Viskosität bei 25 °C im Bereich von 0,5 bis 8 Centistokes (von 0,5 bis 8 $mm^2/s$) aufweisen.

**9.** Zusammensetzung nach einem der vorhergehenden Ansprüche, wobei die acyclischen flüchtigen Silikonöle linear sind und spezieller aus Octamethyltrisiloxan, Decamethyltetrasiloxan und Dodecamethylpentasiloxan und Mischungen davon und spezieller Dodecamethylpentasiloxan ausgewählt sind.

**10.** Zusammensetzung nach einem der vorhergehenden Ansprüche, wobei das Gewichtsverhältnis des acyclischen flüchtigen Silikonöls und der Mischung von β,β-Diphenylacrylat/Salicylat-Filtersubstanzen größer als oder gleich 0,5 ist, weiter bevorzugt größer als oder gleich 0,7 ist, spezieller 0,5 bis 3 beträgt und noch besser 0,7 bis 1,5 beträgt.

**11.** Zusammensetzung nach einem der vorhergehenden Ansprüche, umfassend mindestens 5 Gew.-% Pigmente, weiter bevorzugt 5 bis 25 Gew.-% Pigmente, insbesondere 10 bis 30 Gew.-% und vorzugsweise 10 bis 20 Gew.-% Pigmente, bezogen auf das Gesamtgewicht der Zusammensetzung.

**12.** Zusammensetzung nach einem der vorhergehenden Ansprüche, umfassend mindestens ein mit mindestens einer lipophilen oder hydrophilen Verbindung beschichtetes Pigment, insbesondere hydrophob beschichtete Pigmente von Eisenoxid und/oder von Titandioxid.

**13.** Zusammensetzung nach einem der Ansprüche 1 bis 12 in Form einer wasserfreien Zusammensetzung.

**14.** Zusammensetzung nach einem der Ansprüche 1 bis 12 in Form einer Wasser-in-Öl-Emulsion.

**15.** Zusammensetzung nach einem der vorhergehenden Ansprüche, die in Form einer Grundierung vorliegt.

## Revendications

**1.** Composition, en particulier comprenant un milieu physiologiquement acceptable, en particulier pour le revêtement de matériaux kératiniques, plus particulièrement pour le maquillage et/ou le soin des matériaux kératiniques tels que la peau, contenant au moins :

a) une phase continue huileuse, et
b) au moins un mélange d'agents filtrant les UV organiques liquides comprenant :

(i) au moins un agent filtrant les UV du type β,β-diphénylacrylate, et
(ii) au moins un agent filtrant les UV du type salicylate ; et

c) au moins une huile de silicone volatile non cyclique ; et
d) au moins un pigment ; ladite composition étant :

i) une composition anhydre comprenant une phase huileuse unique, ou

ii) une émulsion eau dans huile.

2. Composition selon la revendication 1, dans laquelle la concentration dudit mélange d'agents filtrant les UV organiques liquides est d'au moins 15 % en poids, et plus préférablement à une concentration d'au moins 20 % en poids, par rapport au poids total de la composition.

3. Composition selon la revendication 1 ou 2, dans laquelle la concentration dudit mélange d'agents filtrant les UV organiques liquides est dans la plage de 15 % à 40 % en poids, et plus préférablement dans la plage de 20 % à 30 % en poids, par rapport au poids total de la composition.

4. Composition selon l'une quelconque des revendications précédentes, dans laquelle l'agent filtrant les UV du type β,β-diphénylacrylate est le composé 2-cyano-3,3-diphénylacrylate de 2-éthylhexyle ou l'octocrylène.

5. Composition selon l'une quelconque des revendications précédentes, dans laquelle l'agent filtrant les UV du type salicylate est choisi parmi :

- l'homosalate,
- le salicylate d'éthylhexyle,
- et des mélanges de ceux-ci.

6. Composition selon l'une quelconque des revendications précédentes, dans laquelle la concentration totale de phase huileuse de la composition de l'invention est de préférence dans la plage de 30 % à 95 % en poids et plus particulièrement dans la plage de 40 % à 70 % en poids par rapport au poids total de la composition.

7. Composition selon l'une quelconque des revendications précédentes, dans laquelle les huiles de silicone volatiles non cycliques sont choisies parmi :

- les silicones linéaires non cycliques de formule (II) :

$$R_3SiO-(R_2SiO)_n-SiR_3 \qquad (II)$$

dans laquelle R, qui peut être identique ou différent, désigne :

- un radical à base d'hydrocarbure saturé ou insaturé, contenant de 1 à 10 atomes de carbone et de préférence de 1 à 6 atomes de carbone, facultativement substitué par un ou plusieurs atomes de fluor ou par un ou plusieurs groupes hydroxyle, ou
- un groupe hydroxyle,

l'un des radicaux R étant éventuellement un groupe phényle,
n est un entier dans la plage de 0 à 8, de préférence dans la plage de 2 à 6 et encore plus préférablement dans la plage de 3 à 5, le composé de silicone de formule (II) ne contenant pas plus de 15 atomes de carbone,

- les silicones ramifiés de formule (III) ou (IV) ci-dessous :

$$R_3SiO-[(R_3SiO)RSiO]-(R_2SiO)x-SiR_3 \qquad (III)$$

$$[R_3SiO]_4Si \qquad (IV)$$

dans lesquelles R, qui peut être identique ou différent, désigne :

- un radical à base d'hydrocarbure saturé ou insaturé, contenant de 1 à 10 atomes de carbone, facultativement substitué par un ou plusieurs atomes de fluor ou par un ou plusieurs groupes hydroxyle, ou
- un groupe hydroxyle,

l'un des radicaux R étant éventuellement un groupe phényle,
x est un entier dans la plage de 0 à 8,

le composé de silicone de formule (III) ou (IV) ne contenant pas plus de 15 atomes de carbone.

**8.** Composition selon l'une quelconque des revendications précédentes, dans laquelle les huiles de silicone volatile non cyclique ont une viscosité à 25 °C dans la plage de 0,5 à 8 centistokes (de 0,5 à 8 $mm^2/s$).

**9.** Composition selon l'une quelconque des revendications précédentes, dans laquelle les huiles de silicone volatile non cycliques sont linéaires, et plus particulièrement choisies parmi l'octaméthyltrisiloxane, le décaméthyltétrasiloxane et le dodécaméthylpentasiloxane, et des mélanges de ceux-ci, et plus particulièrement le dodécaméthylpentasiloxane.

**10.** Composition selon l'une quelconque des revendications précédentes, dans laquelle le rapport en poids d'huile de silicone volatile non cyclique / mélange d'agents filtrants de type β,β-diphénylacrylate/salicylate est supérieur ou égal à 0,5, plus préférablement supérieur ou égal à 0,7, plus particulièrement de 0,5 à 3 et encore plus préférablement de 0,7 à 1,5.

**11.** Composition selon l'une quelconque des revendications précédentes, comprenant au moins 5 % en poids de pigments, plus préférablement de 5 % à 25 % en poids de pigments, en particulier de 10 % à 30 % en poids et de préférence de 10 % à 20 % en poids de pigments, par rapport au poids total de ladite composition.

**12.** Composition selon l'une quelconque des revendications précédentes, comprenant au moins un pigment revêtu avec au moins un composé lipophile ou hydrophobe, en particulier des pigments à revêtement hydrophobe d'oxyde de fer et/ou de dioxyde de titane.

**13.** Composition selon l'une quelconque des revendications 1 à 12, sous la forme d'une composition anhydre.

**14.** Composition selon l'une quelconque des revendications 1 à 12, sous la forme d'une émulsion eau dans huile.

**15.** Composition selon l'une quelconque des revendications précédentes, qui est sous la forme d'un fond de teint.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010045163 A
- US 20110033512 A
- WO 2007148293 A
- WO 2013078197 A
- US 20130315846 A
- FR 2861593
- DE 102008012457
- FR 0853634
- EP 0955039 A
- US 2004175338 A
- EP 847752 A
- US 4578266 A
- JP H07196946 B
- US 5725882 A
- US 5209924 A
- US 4972037 A
- US 4981903 A
- US 4981902 A
- US 5468477 A
- US 5219560 A
- EP 0388582 A
- US 5246694 A
- EP 0486135 A
- JP H0586984 B
- FR 2679771
- EP 1184426 A
- US 5236986 A
- US 5412004 A
- US 5837793 A
- US 5811487 A
- WO 2004024798 A
- EP 0392883 A
- US 5237071 A
- US 5166355 A
- GB 2303549 A
- DE 19726184
- EP 893119 A
- US 5624663 A
- EP 669323 A
- US 2463264 A
- EP 0832642 A
- EP 1027883 A
- EP 1300137 A
- DE 10162844
- WO 9304665 A
- US 4195999 A
- WO 2004006878 A
- WO 2008090066 A
- WO 2011113718 A
- WO 2009027258 A
- WO 2013010590 A
- WO 2013011094 A
- WO 2013011480 A
- WO 2007071584 A
- WO 2009063392 A
- US 6225467 B
- WO 2004085412 A
- WO 06035000 A
- WO 06034982 A
- WO 06034991 A
- WO 06035007 A
- WO 2006034992 A
- WO 2006034985 A
- EP 0841341 A
- EP 0518773 A
- US 5538793 A


### Non-patent literature cited in the description


- **VAN DE HULST, H.C.** Light Scattering by Small Particles. Wiley, 1957
- **WITUCKI.** A silane primer, Chemistry and applications of alkoxy silanes. *Journal of Coatings Technology,* 1993, vol. 65 (822), 57-60
- **C.M. HANSEN.** The three-dimensional solubility parameters. *J. Paint Technol.,* 1967, vol. 39, 105
- The HLB system. A time-saving guide to Emulsifier Selection. ICI Americas Inc, 1984
- *IP COM JOURNAL,* 23 February 2009
- *IP COM JOURNAL,* 29 April 2009
- *IP COM JOURNAL,* 12 November 2009
- *IP COM Journal,* 04 March 2004
- *CHEMICAL ABSTRACTS,* 919803-06-8
- Symmetrical Triazine Derivatives. *IP.COM IPCOM000031257 Journal, INC, West Henrietta, NY, US,* 20 September 2004
- **B.L. DIFFEY.** *J. Soc. Cosmet. Chem.,* 1989, vol. 40, 127-133